# EUROPEAN PATENT SPECIFICATION

(11) **EP 3 538 889 B1**
(45) Date of publication and mention of the grant of the patent: **28.09.2022**
(21) Application number: 17891415.6
(22) Date of filing: 26.12.2017
(51) Int. Cl.: G01N 33/532, B82Y 15/00

(54) **A METHOD OF DETECTING THE PRESENCE OF DIFFERENT TARGET ANALYTES AND RELATED KITS THEREOF**
VERFAHREN ZUR ERKENNUNG DES VORHANDENSEINS VON VERSCHIEDENEN ZIELANALYTEN UND ZUGEHÖRIGE KITS DAFÜR
PROCÉDÉ DE DÉTECTION DE LA PRÉSENCE DE DIFFÉRENTS ANALYTES CIBLES ET KITS ASSOCIÉS

(30) Priority: 12.01.2017 SG 10201700257X
(43) Date of publication of application: 18.09.2019
(73) Proprietor: Agency for Science, Technology and Research, Singapore 138632 (SG)
(72) Inventor: YING, Jackie Y., Singapore 138669 (SG); ZHANG, Yi, Singapore 138669 (SG); YU, Yue, Singapore 138669 (SG)
(74) Representative: EIP
(86) International application number: PCT/SG2017/050645
(87) International publication number: WO 2018/132062

(56) References cited:
- EP-A1- 2 503 337
- EP-A1- 2 636 469
- EP-A1- 2 853 894
- EP-A2- 0 227 173
- WO-A1-2017/138946
- WO-A2-2008/070865
- CN-A- 101 852 799
- US-A1- 2005 250 141
- US-A1- 2009 305 290
- US-A1- 2017 234 866
- KRISTOFER J. HAUSHALTER ET AL: "Multiplex Flow Assays", ACS OMEGA, vol. 1, no. 4, 31 October 2016 (2016-10-31), pages 586-599, XP055679935, ISSN: 2470-1343, DOI: 10.1021/acsomega.6b00188
- YEN, C.-W. ET AL.: 'Multicolored silver nanoparticles for multiplexed disease diagnostics: distinguishing dengue, yellow fever, and Ebola viruses' LAB ON A CHIP vol. 15, no. 7, 17 March 2015, pages 1638 - 1641, XP055359648
- LEE, S. ET AL.: 'Two-Color Lateral Flow Assay for Multiplex Detection of Causative Agents Behind Acute Febrile Illnesses' ANALYTICAL CHEMISTRY vol. 88, no. 17, 04 August 2016, pages 8359 - 8363, XP055517028

### SEQUENCE LISTING

<110> Agency for Science, Technology and Research
<120> A METHOD OF DETECTING THE PRESENCE OF DIFFERENT TARGET ANALYTES AND RELATED KITS THEREOF
<130> 1702371
<160> 24
<170> PatentIn version 3.5
<210> 1
   <211> 42
   <212> DNA
   <213> Artificial Sequence
<220>
   <223> oligonucleotide seqeunce
<400> 1
   ccagacgttc cgttggtagg tcactttgac tattcgcgca cc 42
<210> 2
   <211> 39
   <212> DNA
   <213> Artificial Sequence
<220>
   <223> oligonucleotide sequence
<400> 2
   ccatcatcgc cacgtcggac tcggcgtctg ctttttgtg 39
<210> 3
   <211> 18
   <212> DNA
   <213> Artificial Sequence
<220>
   <223> oligonucleotide seqeunce
<400> 3
   cagccacaca cctgtgaa 18
<210> 4
   <211> 19
   <212> DNA
   <213> Artificial Sequence
<220>
   <223> oligonucleotide seqeunce
<400> 4
   tccgtcgagg catcgttag 19
<210> 5
   <211> 24
   <212> DNA
   <213> Artificial Sequence
<220>
   <223> oligonucleotide seqeunce
<400> 5
   aaatcctgtc gccctacaca gcgg 24
<210> 6
   <211> 20
   <212> DNA
   <213> Artificial Sequence
<220>
   <223> oligonucleotide seqeunce
<400> 6
   caccccgcga cgggacgccg 20
<210> 7
   <211> 39
   <212> DNA
   <213> Artificial Sequence
<220>
   <223> oligonucleotide sequence
<400> 7
   ccctggtacg tgacgtgtac gagtatggag ggtgtcgcg 39
<210> 8
   <211> 39
   <212> DNA
   <213> Artificial Sequence
<220>
   <223> oligonucleotide sequence
<400> 8
   aaatgcttcc ctgctggtgc ccgccgagtt cgatctggt 39
<210> 9
   <211> 18
   <212> DNA
   <213> Artificial Sequence
<220>
   <223> oligonucleotide sequence
<400> 9
   tcagcccatc ctccttcg 18
<210> 10
   <211> 18
   <212> DNA
   <213> Artificial Sequence
<220>
   <223> oligonucleotide sequence
<400> 10
   gcccacctct acccacaa 18
<210> 11
   <211> 22
   <212> DNA
   <213> Artificial Sequence
<220>
   <223> oligonucleotide sequence
<400> 11
   cacgtctttg gggacggcgg ct 22
<210> 12
   <211> 25
   <212> DNA
   <213> Artificial Sequence
<220>
   <223> oligonucleotide sequence
<400> 12
   atctgggacc gcgccgcgga gacat 25
<210> 13
   <211> 40
   <212> DNA
   <213> Artificial Sequence
<220>
   <223> oligonucleotide sequence
<400> 13
   ctcccactgg tacgtcaagt ggagggtcaa aaaccctggc 40
<210> 14
   <211> 40
   <212> DNA
   <213> Artificial Sequence
<220>
   <223> oligonucleotide sequence
<400> 14
   ttcctcaaca tccccgacat cgtacccgat gggggatacc 40
<210> 15
   <211> 19
   <212> DNA
   <213> Artificial Sequence
<220>
   <223> oligonucleotide sequence
<400> 15
   ccaatacgac caccggatc 19
<210> 16
   <211> 20
   <212> DNA
   <213> Artificial Sequence
<220>
   <223> oligonucleotide sequence
<400> 16
   tggcttcgtc tcgaggattt 20
<210> 17
   <211> 20
   <212> DNA
   <213> Artificial Sequence
<220>
   <223> oligonucleotide sequence
<400> 17
   cgaaatgtag gatataaagg 20
<210> 18
   <211> 23
   <212> DNA
   <213> Artificial Sequence
<220>
   <223> oligonucleotide sequence
<400> 18
   catccttata ttcaaaagta gct 23
<210> 19
   <211> 41
   <212> DNA
   <213> Artificial Sequence
<220>
   <223> oligonucleotide sequence
<400> 19
   cgacgaccct tctttttcct caagccaaac ctaaaaccag c 41
<210> 20
   <211> 44
   <212> DNA
   <213> Artificial Sequence
<220>
   <223> oligonucleotide sequence
<400> 20
   tctcgataaa gtctctacag agaccttttt ccagatcttc acgc 44
<210> 21
   <211> 18
   <212> DNA
   <213> Artificial Sequence
<220>
   <223> oligonucleotide sequence
<400> 21
   gtgaagcaaa acgtagcg 18
<210> 22
   <211> 18
   <212> DNA
   <213> Artificial Sequence
<220>
   <223> oligonucleotide sequence
<400> 22
   ctgactacgt agacgctc 18
<210> 23
   <211> 17
   <212> DNA
   <213> Artificial Sequence
<220>
   <223> oligonucleotide sequence
<400> 23
   agcggctgct cgccttt 17
<210> 24
   <211> 25
   <212> DNA
   <213> Artificial Sequence
<220>
   <223> oligonucleotide sequence
<400> 24
   accgttgata ttacttgtgc cgaag 25

## Description

### TECHNICAL FIELD

The present disclosure relates broadly to an in vitro method of detecting the presence of different target analytes and related kits thereof.

### BACKGROUND

Paper or membrane-based diagnostic platform is a widely adopted technology due to its low cost, simple operation, and equipment free signal readout, leading to a high market demand. An example is lateral flow assay (LFA), which is a well-established paper-based platform for a wide variety of applications, including point-of-care diagnostics for fertility issues, metabolic syndromes and organ functions. Such diagnostic platforms including LFA often rely on ligand-receptor interaction to recognise the target of interest. Capture agents, which are usually antibodies or high affinity bind proteins (such as streptavidin), form a capture-detector pair to immobilize the target molecules at the test zone and tag them with reporter conjugates. Many types of reporter conjugates, including fluorescent molecules, quantum dots, upconverting phosphor, enzymes and colloidal particles, have been utilised in diagnostic platforms including LFA.

Multiplexed diagnostic platform, in which a multitude of targets are analysed on a single platform, is a highly desired configuration as it would considerably increase the diagnostic efficiency and reduce the required sample volume. Currently, multiplexing techniques used in paper or membrane-based diagnostic platforms including LFAs are limited as they are typically realized by spatially separating test lines along the test strip (e.g. single parameter). Such multiplexing capability is therefore limited by space restrictions. Other existing coding systems that use more than one parameter for detection are incompatible with diagnostic platforms such as LFA due to the large size of the barcode and complicated instrumentation for decoding.

In view of the above, there is thus a need to address or at least ameliorate one or more of the above problems.

### SUMMARY

The invention is set out in the appended set of claims.

In one aspect, there is provided an in vitro method of detecting the presence of different target analytes in a sample, the method comprising: providing the sample and a plurality of reporter particles having ω different colours to a substrate, the substrate having α assigned spatial regions; and determining, on the substrate, the presence of a colour arising from the reporter particles in each of α assigned spatial regions on the substrate, wherein each of the assigned spatial regions on the substrate is configured to immobilize a target analyte or a group of target analytes that is different from the other assigned spatial regions, wherein reporter particles of each different colour are configured to couple to a target analyte that is different from that of the reporter particles of the other different colours, and wherein α is an integer that is at least two and ω is an integer that is at least three, wherein the reporter particles are configured to give rise to a secondary colour when reporter particles of two or more different colours are colocalized in the same spatial region, wherein the secondary colour resulting from the colocalization of the reporter particles of two or more different colours from the ω different colours is distinct from another secondary colour resulting from the colocalization of reporter particles of other combinations of colours from the ω different colours, wherein the presence of a secondary colour arising from the colocalization of reporter particles in an assigned spatial region is indicative of the presence of at least two different target analytes in the sample.

In one embodiment, ω is three.

In one embodiment, each of ω different colours is a colour selected from the group consisting of: a colour with absorbance wavelength of from 550 nm to 570 nm, a colour with absorbance wavelength of from 400 nm to 420 nm and a colour with absorbance wavelength of from 610 nm to 630 nm.

In one embodiment, each of ω different colours is a colour selected from the group consisting of: yellow, magenta and cyan, and when combined with one of more of the other ω different colours, is configured to give rise to a secondary colour selected from: blue, green, red and black.

In one embodiment, the analyte comprises a polynucleotide and the method further comprises subjecting the sample to an amplification reaction configured to amplify the polynucleotide prior to the providing step.

In one embodiment, the analyte comprises a polynucleotide and the method further comprises contacting the sample with a labelled oligonucleotide for hybridizing to at least a portion of the polynucleotide or its amplicons thereof, the labelled oligonucleotide being configured to couple to the reporter particles of at least one of ω different colours.

In one embodiment, the oligonucleotide comprises a primer.

In one embodiment, the step of providing the sample and the plurality of reporter particles having ω different colours to a substrate comprises first providing the sample to the substrate and subsequently providing the plurality of reporter particles having ω different colours to the substrate.

In one embodiment, the reporter particles of each of ω different colours have a single localized surface plasmon resonance (LSPR) absorption peak.

In one embodiment, the reporter particles comprise one or more of the following selected from the group consisting of: solid gold nanoparticles, solid silver nanoparticles with gold core and hollow gold nanoshells.

In one embodiment, the method further comprises: assigning a code to each spatial region based on the colour at the spatial region; combining the code assigned to each spatial region to obtain a combined code, wherein the combined code is used to determine the presence of different target analytes in the sample; and optionally comparing the combined code with a list of predetermined codes to determine the presence of different target analytes in the sample.

In one aspect, there is provided a kit for detecting different target analytes in a sample, the kit comprising: a substrate that comprises α assigned spatial regions, each spatial region being configured to immobilize a target analyte or a group of target analytes that is different from the other assigned spatial regions; and a plurality of reporter particles having ω different colours or precursors thereof, wherein the ω different colours are such that a secondary colour resulting from a combination of two or more of the ω different colours is distinct from other combinations of the ω different colours, wherein the reporter particles of each different colour are configured to couple to a target analyte that is different from that of the reporter particles of the other different colours, and wherein α is an integer that is at least two and ω is an integer that is at least three.

In one embodiment of the kit, ω is three.

In one embodiment of the kit, each of ω different colours is a colour selected from the group consisting of: a colour with absorbance wavelength of from 550 nm to 570 nm, a colour with absorbance wavelength of from 400 nm to 420 nm and a colour with absorbance wavelength of from 610 nm to 630 nm.

In one embodiment of the kit, each of ω different colours is a colour selected from the group consisting of: yellow, magenta and cyan, and when combined with one of more of the other ω different colours, is configured to give rise to a secondary colour selected from: blue, green, red and black.

In one embodiment, the kit further comprises an oligonucleotide for hybridizing to at least a portion of a polynucleotide of the target analyte.

In one embodiment of the kit, the oligonucleotide comprises a labelled oligonucleotide that is configured to couple to the reporter particles of at least one of ω different colours.

In one embodiment of the kit, the reporter particles of each of ω different colours have a single localized surface plasmon resonance (LSPR) absorption peak.

In one embodiment of the kit, the reporter particles comprise one or more of the following selected from the group consisting of: solid gold nanoparticles, solid silver nanoparticles with gold core, hollow gold nanoshells and any combinations thereof.

In one embodiment, the kit further comprises:
a list of predetermined codes as reference for determining the presence of different target analytes in the sample.

### DEFINITIONS

As used herein, the term "analyte" broadly refers to a substance to be detected.

As used herein, the term "sample", unless otherwise stated, refers to a representative portion of a larger object/matter. A sample may be a "test sample" or a "control sample". A "test sample" is usually a sample that may contain or is suspected to contain one or more target analytes and it is to be distinguished from "control sample", which is usually a sample known to contain or lack one or more target analytes.

The term "nano" as used herein is to be interpreted broadly to include dimensions less than about 1000 nm. Exemplary sub-ranges that fall within the term include but are not limited to the ranges of less than about 900 nm, less than about 800 nm, less than about 700 nm, less than about 600 nm, less than about 500 nm, less than about 400 nm, less than about 300 nm, less than about 200 nm, less than about 100 nm, less than about 50 nm, less than about 20 nm, or less than about 10 nm. Accordingly, "nanoparticles" or "nanostructures" as used herein broadly refer to any particles or structures of sizes that are less than about 1000 nm, including but not limited to nanocomposites. Further, the term "nanoparticles" encompasses but is not limited to nanoparticles in the form of colloidal nanoparticles.

The term "particle" as used herein broadly refers to a discrete entity or a discrete body. The particle described herein can include an organic, an inorganic or a biological particle. The particle used described herein may also be a macro-particle that is formed by an aggregate of a plurality of sub-particles or a fragment of a small object. The particle of the present disclosure may be spherical, substantially spherical, or non-spherical, such as irregularly shaped particles or ellipsoidally shaped particles. The term "size" when used to refer to the particle broadly refers to the largest dimension of the particle. For example, when the particle is substantially spherical, the term "size" can refer to the diameter of the particle; or when the particle is substantially non-spherical, the term "size" can refer to the largest length of the particle.

The term "couple" broadly refers to a stable association between two substances brought about by any chemical, physical, or physicochemical interactions, such as by covalent bond, hydrogen bond, electrostatic interaction, polar attraction, van der Waals' attraction, hydrophobic interaction or adsorption. Unless otherwise stated, the term is intended to cover both a direct association between two substances, for example, the direct binding/conjugation of an antibody to an antigen on a protein, and an indirect association between two substances through one or more intermediate means, for example, an association between an antibody and a polynucleotide by way of one or more oligonucleotides and/or labels. Accordingly, "coupled", when used in relation to two substances, refer to two substances being associated by any such indirect or indirect means.

The term "label" as used herein broadly refers to any substance that can be coupled to or incorporated in a target analyte to aid the detection of the target analyte. Although in some cases a "label" may be directly detected, it need not be directly detected as it may also be indirectly detected via a proxy, for example, it may be indirectly detected by a colour arising from a coloured reporter particle configured to couple to the label.

The term "associated with", used herein when referring to two elements refers to a broad relationship between the two elements. The relationship includes, but is not limited to a physical, a chemical or a biological relationship. For example, when element A is associated with element B, elements A and B may be directly or indirectly attached to each other or element A may contain element B or vice versa.

The term "and/or", e.g., "X and/or Y" is understood to mean either "X and Y" or "X or Y" and should be taken to provide explicit support for both meanings or for either meaning.

Further, in the description herein, the word "substantially" whenever used is understood to include, but not restricted to, "entirely" or "completely" and the like. In addition, terms such as "comprising", "comprise", and the like whenever used, are intended to be non-restricting descriptive language in that they broadly include elements/components recited after such terms, in addition to other components not explicitly recited. Terms such as "consisting", "consist", and the like, may in the appropriate context, be considered as a subset of terms such as "comprising", "comprise", and the like. Therefore, in embodiments disclosed herein using the terms such as "comprising", "comprise", and the like, it will be appreciated that these embodiments provide teaching for corresponding embodiments using terms such as "consisting", "consist", and the like. Further, terms such as "about", "approximately" and the like whenever used, typically means a reasonable variation, for example a variation of +/- 5% of the disclosed value, or a variance of 4% of the disclosed value, or a variance of 3% of the disclosed value, a variance of 2% of the disclosed value or a variance of 1% of the disclosed value.

Furthermore, in the description herein, certain values may be disclosed in a range. The values showing the end points of a range are intended to illustrate a preferred range. Whenever a range has been described, it is intended that the range covers and teaches all possible sub-ranges as well as individual numerical values within that range. That is, the end points of a range should not be interpreted as inflexible limitations. For example, a description of a range of 1% to 5% is intended to have specifically disclosed sub-ranges 1% to 2%, 1% to 3%, 1% to 4%, 2% to 3% etc., as well as individually, values within that range such as 1%, 2%, 3%, 4% and 5%. The intention of the above specific disclosure is applicable to any depth/breadth of a range.

Additionally, when describing some embodiments, the disclosure may have disclosed a method and/or process as a particular sequence of steps. However, unless otherwise required, it will be appreciated that the method or process should not be limited to the particular sequence of steps disclosed. Other sequences of steps may be possible. The particular order of the steps disclosed herein should not be construed as undue limitations. Unless otherwise required, a method and/or process disclosed herein should not be limited to the steps being carried out in the order written. The sequence of steps may be varied and still remain within the scope of the disclosure.

### DESCRIPTION OF EMBODIMENTS

Exemplary, non-limiting embodiments of a method of detecting the presence of different target analytes and related kits thereof are disclosed hereinafter.

In one aspect, there is provided a method for determining the presence of different target analytes in a sample, the method comprising: detecting in the sample a first parameter that is being assigned α possible variables and a second parameter that is being assigned β possible variables to determine the presence of the different target analytes, wherein the first parameter is different from the second parameter, wherein α is an integer that is at least two and β is an integer that is at least three.

Embodiments of the method described herein may be implemented in a paper or membrane-based diagnostic platform such as LFA but is not limited as such. Advantageously, embodiments of the method employ at least two different parameters to improve the multiplexing capability of a diagnostic assay.

In one embodiment, α is an integer that is at least two, at least three, at least four, at least five or at least six or at least seven and β is an integer that is at least two, at least three, at least four, at least five, at least six or at least seven. Advantageously, embodiments of the method are able to provide a large number of combinations of variables, thereby allowing for the detection of a large number of analytes in a single test/assay or on a single platform.

In various embodiments, the parameter is selected from the group consisting of: spatial position, colour, colour intensity, hue and size of coloured area. The first parameter comprises a spatial parameter. Accordingly, in an embodiment, the assigned α possible variables of the first parameter comprise α different assigned spatial positions on a substrate. The second parameter comprises a colour parameter. Accordingly, in an embodiment, the assigned β possible variables of the second parameter comprise β different assigned colours.

In one embodiment, the method comprises contacting the sample with a substrate that comprises α different assigned spatial regions, each of the spatial region configured to immobilise a different group of target analytes.

In one embodiment, the method further comprises contacting the substrate with reporter molecules/particles having different colours, the reporter molecules/particles tagged with a colour selected from the β different assigned colours and the reporter particles of each different colour are configured to conjugate to a different analyte. In one embodiment, the method further comprises incubating the sample with reporter molecules/particles having different colours to form a mixture, the reporter molecules/particles tagged with a colour selected from the β different assigned colours and the reporter particles of each different colour reporter molecule/particle are configured to couple to a different analyte; and contacting the mixture with a substrate. The reporter molecules have ω different colours, the ω different colours being selected from the β different assigned colours.

Provided is a method of detecting the presence of different target analytes in a sample, the method comprising: providing the sample and a plurality of reporter particles having ω different colours to a substrate, the substrate having α assigned spatial regions; and determining, on the substrate, the presence of a colour arising from the reporter particles in each of α assigned spatial regions on the substrate, wherein each of the assigned spatial regions on the substrate is configured to immobilize a target analyte that is different from the other assigned spatial regions, wherein reporter particles of each different colour are configured to couple to a target analyte that is different from the reporter particles of the other different colours, and wherein α is an integer that is at least two and ω is an integer that is at least three.

Advantageously, embodiments of the method employing both spatial position and colour may be able to achieve a high level of multiplexing, thereby significantly improving the multiplexing capability and extending the applicability of the diagnostic platforms such as LFAs to more complicated analysis. Further, to analyse the same number of target analytes, embodiments of the method may require less test zones on the diagnostic platforms such as LFAs, thereby reducing cost and time required to manufacture the diagnostic platforms.

The reporter particles are configured to give rise to a secondary colour when reporter particles of two or more different colours are colocalized in the same spatial region, wherein the secondary colour resulting from the colocalization of the reporter particles of two or more different colours from the ω different colours is distinct from another secondary colour resulting from the colocalization of reporter particles of other combinations of colours from the ω different colours. In various embodiments, it may be understood that the secondary colour resulting from the colocalization of reporter particles/molecules of two or more different colours from the ω different colours is distinct/different from each of ω different colours or at least can be easily distinguished from each of ω different colours either visually or otherwise.

In one embodiment, ω is three. In one embodiment, ω is less than β. In some embodiments, ω represents the number of different primary colours of the reporter molecules/particles while β represents the total number of the different primary colours and the possible secondary colours derived from the primary colours. Accordingly, in one embodiment, where there are reporter particles having three different colours, there are at least four, at least five, at least six or at least seven possible assigned colours. Advantageously, embodiments of the method is able to identify a large number of combination of analytes with a small input.

In one embodiment, the parameter comprises a visually perceptible parameter, wherein the visually perceptible parameter is visually perceptible to a human eye.

Accordingly, in one embodiment, each of ω different colours is visually distinct/distinguishable/ different/unique to the human eye. In a further embodiment, the secondary colour resulting from the colocalization of reporter particles/molecules of two or more different colours from ω different colours is visually distinct/distinguishable/ different/unique from each of ω different colours to the human eye. In yet a further embodiment, the secondary colour resulting from the colocalization of reporter particles/molecules of two or more different colours from ω different colours is visually distinct/ distinguishable/different/unique from another secondary colour resulting from the colocalization of reporter particles/molecules of other combinations of colours from the ω different colours to the human eye.

In one embodiment, the α assigned spatial regions comprises α distinct spatial regions. The distinct spatial regions may be separated from one another by one or more regions on the substrate that do not partake in the detection, for example these regions that do not partake in the detection may be substantially inert and/or do not result in a ligand-receptor interaction with the analytes and/or reporter particles. For example, these regions do not comprise a capturing agent for immobilising a target analyte. In a further embodiment, the α distinct spatial regions comprises α visually distinct spatial regions to the human eye, preferably with a naked or unaided human eye.

Advantageously, embodiments of the method do not require use of elaborate/automated/electronic/electrical devices, equipment or instruments for readout. Embodiments of the method do not require an artificial power source and may be carried out manually. Embodiments of the method are therefore substantially equipment-free, easy to implement, convenient, low cost, efficient and suitable as a point-of-care diagnostic or a home diagnostic.

In various embodiments, each of ω different colours is a colour selected from the group consisting of: a colour with absorbance wavelength of from about 550 nm to about 570 nm, a colour with absorbance wavelength of from about 400 nm to about 420 nm and a colour with absorbance wavelength of from about 610 nm to about 630 nm. In various embodiments, each of ω different colours is a colour selected from the group consisting of: a colour with absorbance wavelength of from about 560 nm to about 565 nm, a colour with absorbance wavelength of from about 405 nm to about 410 nm and a colour with absorbance wavelength of from about 615 nm to about 620 nm. In various embodiments, each of ω different colours is a colour selected from the group consisting of: a colour with absorbance wavelength of about 563 nm, a colour with absorbance wavelength of about 407 nm and a colour with absorbance wavelength of with absorbance wavelength of about 617 nm. In various embodiments, each of ω different colours is a colour selected from the group consisting of: yellow, magenta and cyan.

In various embodiments, the β possible assigned colours comprises a colour selected from the group consisting of: a colour with absorbance wavelength of from about 550 nm to about 570 nm, a colour with absorbance wavelength of from about 555 nm to about 565 nm, a colour with absorbance wavelength of from about 560 nm to about 565 nm, a colour with absorbance wavelength of about 563 nm, a colour with absorbance wavelength of from about 400 nm to about 420 nm, a colour with absorbance wavelength of from about 405 nm to about 415 nm, a colour with absorbance wavelength of from about 405 nm to about 410 nm, a colour with absorbance wavelength of about 407 nm, a colour with absorbance wavelength of from about 610 nm to about 630 nm, a colour with absorbance wavelength of from about 615 nm to about 625 nm, a colour with absorbance wavelength of from about 615 nm to about 620 nm or a colour with absorbance wavelength of about 617 nm. In various embodiments, the β possible assigned colours comprises a colour selected from the group consisting of: cyan, magenta, yellow, blue, green, red, black and optionally no visible/detectable colour. It may be appreciated that in various embodiments the colours refer to the hue that is perceptible to the user/operator. Accordingly, variations of tint, tone, and/or shades of the same hue may still be considered as the same colour as long as the user/operator still perceives such tint, tone, and/or shades to belong to the same hue. For example, light yellow and dark yellow may both be considered as yellow in general by the user/operator.

In various embodiments, the combination or the colocalization in a spatial region of two or more colours selected from the group consisting of: cyan, magenta and yellow result in a secondary colour that is visible to the human eye and that is selected from the group consisting of: blue, green, red and black. Accordingly, in various embodiments, the secondary colour is selected from the group consisting of: blue, green, red and black. In one embodiment therefore, each of ω different colours is a colour selected from the group consisting of: yellow, magenta and cyan, and when combined with one or more of the other ω different colours, is configured to give rise to a secondary colour selected from: blue, green, red and black. Therefore, in various embodiments, the primary and secondary colour are based on a subtractive colour system which subtract wavelengths from visible white light and reflect the other wavelengths of light to visually give the primary and secondary colour.

Advantageously, in various embodiments, when the colours are in accordance with the colours disclosed herein, the colours are visually distinct to a human eye, preferably a naked or unaided healthy human eye.

In one embodiment, the presence of a colour arising from the reporter particles in an assigned spatial region is indicative of the presence of at least one target analyte in the sample. The presence of a secondary colour arising from the colocalization reporter particles in an assigned spatial region is indicative of the presence of at least two different target analytes in the sample. In a particular embodiment, the presence of a single colour selected from the group consisting of: yellow, magenta and cyan in an assigned spatial region is indicative of the presence of one analyte in the spatial region. In a particular embodiment, the presence of a single secondary colour selected from the group consisting of: blue, green, red in an assigned spatial region is indicative of the presence of two different analytes in the spatial region. In a particular embodiment, the presence of a secondary colour of black in an assigned spatial region is indicative of the presence of three different analytes in the spatial region. In an embodiment, the absence of a visible/detectable colour in an assigned spatial region is indicative of the absence of one or more target analytes in the sample.

In one embodiment, the presence of a colour arising from the reporter particles in two or more assigned spatial regions is indicative of the presence of two or more target analytes in the sample. Likewise, the presence of a colour arising from the reporter particles in three or more assigned spatial regions may be indicative of the presence of three or more target analytes in the sample and so on and so forth.

In various embodiments, the target analyte comprises at least one amino acid, at least one peptide, at least one polypeptide, at least one protein, at least one nucleotide, at least one polynucleotide, at least one nucleic acid, at least one DNA, at least one RNA, at least one lipid, at least one glycolipid, at least one carbohydrate, at least one hormone, at least one cell, at least one microoganism, at least one bacterial pathogen, at least one bacterial DNA, at least one bacterial RNA, at least one viral pathogen, at least one viral DNA, at least one viral RNA, at least one fragment or at least one subtype of any of the above substances, at least one chemical compound, at least one organic compound, at least one steroid, at least one vitamin, at least one anaesthetic, at least one drug, at least one drug intermediate, at least one pharmaceutical, at least one toxin, at least one pesticide, at least one heavy metal, at least one metal, at least one metal ion, at least one metabolite of any one of the above substances or any combinations thereof. In some embodiments, the polynucleotide or the nucleic acid comprises a double stranded polynucleotide or nucleic acid. In some embodiments, the analyte comprises a biological material. In some embodiments, the analyte comprises a natural material, for example, naturally occurring biological material. In other embodiments, the analyte comprises a synthetic material, for example, pesticides.

Accordingly, in some embodiments, the sample comprises or is suspected to comprise at least one amino acid, at least one peptide, at least one polypeptide, at least one protein, at least one nucleotide, at least one polynucleotide, at least one nucleic acid, at least one DNA, at least one RNA, at least one lipid, at least one glycolipid, at least one carbohydrate, at least one hormone, at least one cell, at least one microoganism, at least one bacterial pathogen, at least one bacterial DNA, at least one bacterial RNA, at least one viral pathogen, at least one viral DNA, at least one viral RNA, at least one fragment or at least one subtype of any of the above substances, at least one chemical compound, at least one organic compound, at least one steroid, at least one vitamin, at least one anaesthetic, at least one drug, at least one drug intermediate, at least one pharmaceutical, at least one toxin, at least one pesticide, at least one heavy metal, at least one metal, at least one metal ion, at least one metabolite of any one of the above substances or any combinations thereof.

In some embodiments, the method comprises obtaining a sample. In some embodiments, the method comprises processing a sample prior to its provision to the substrate. Methods of processing different types of samples known in the art may be employed prior to their provision to the substrate. For example, methods of processing a biological sample may involve cell lysis, extraction of DNA and/or RNA, disintegration and/or dissolving of sample, purification and sample concentration. In one embodiment, the method further comprises substantially dissolving/solubilizing the sample in a solvent to obtain a fluid sample. In one embodiment therefore, the sample comprises a fluid sample.

In some embodiments, wherein the analyte comprises a polynucleotide or a nucleic acid, preparing the sample comprises subjecting the sample to an amplification condition suitable for amplifying the nucleic acid analyte to a detectable level. In some embodiments, the analyte comprises a polynucleotide/nucleic acid, and the method further comprises subjecting the sample to an amplification reaction configured to amplify the polynucleotide /nucleic acid prior to the providing step. Amplification reactions known in the art may be employed. The amplification reactions may include but are not limited to polymerase chain reaction (PCR), ligase chain reaction (LCR), loop mediated isothermal amplification (LAMP), nucleic acid sequence based amplification (NASBA), self-sustained sequence replication (3SR), rolling circle amplification (RCA) or any other process whereby one or more copies of a particular polynucleotide sequence or nucleic acid sequence may be generated from a polynucleotide template sequence or nucleic acid template sequence. In one embodiment, the amplification reaction comprises an isothermal amplification reaction. In one embodiment, the amplification reaction comprises LAMP. As may be appreciated, LAMP has high amplification efficiency as compared to, for example, conventional PCR. Embodiments of the method comprising using LAMP for amplification may therefore require a shorter time to complete as compared to, for example, a method using conventional PCR for amplification and therefore embodiments of the method are particularly suitable as point-of-care diagnostics.

In some embodiments, wherein the analyte comprises a polynucleotide or a nucleic acid, preparing the sample comprises subjecting the sample to a labelling procedure configured to couple the polynucleotide, the nucleic acid and/or their amplicons thereof to a label, the label being configured to couple to reporter particles of at least one of ω different colours. In one embodiment, the labelling procedure comprises contacting the sample with an oligonucleotide for hybridizing to at least a portion of the polynucleotide, the nucleic acid and/or their amplicons thereof, the oligonucleotide being coupled to the label. In one embodiment therefore, the analyte comprises a polynucleotide/nucleic acid and the method further comprises contacting the sample with a labelled oligonucleotide for hybridizing to at least a portion of the polynucleotide/nucleic acid or its amplicons thereof, the labelled oligonucleotide being configured to couple to the reporter particles of at least one of ω different colours.

In one embodiment, the oligonucleotide comprises a primer. In one embodiment therefore, the labelling procedure takes place simultaneously with an amplification reaction, the method comprising contacting the sample with a labelled primer suitable for amplifying the polynucleotide or the nucleic acid. In one embodiment, the labelling procedure comprises contacting the sample with two different oligonucleotides, wherein each oligonucleotide is configured to hybridize to one of the two complementary strands of a double stranded polynucleotide or the nucleic acid, the oligonucleotides each being coupled to a label to eventually obtain a dually labelled double stranded polynucleotide or nucleic acid.

In various embodiments, the oligonucleotide comprises one or more sequences selected from the group consisting of: SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24 or a sequence sharing at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98% or at least about 99% identity with any one of the above sequences.

In one embodiment, the label is capable of interacting with or coupling to the reporter particles. In one embodiment, the label is capable of interacting with or coupling to a capturing agent which may be disposed on the substrate. In a preferred embodiment, each label is capable of interacting with or coupling to reporter particles of one of ω different colours or one of the capturing agents which may be disposed on the substrate. In one embodiment, the label comprises a ligand or a hapten. In various embodiments, the ligand or hapten is selected from the group consisting of: biotin, digoxigenin (DIG), dinitrophenol (DNP), fluorescein such as 6-FAM or FITC, Texas red, 5-carboxytetramethylrhodamine (TAMRA), or the like and any combinations thereof.

In one embodiment, the step of providing the sample and a plurality of reporter particles having ω different colours to a substrate comprises providing the sample and the plurality of reporter particles having ω different colours separately or sequentially to the substrate. In a particular embodiment, the step of providing the sample and a plurality of reporter particles having ω different colours to a substrate comprises first providing the sample to the substrate and subsequently providing the plurality of reporter particles having ω different colours to the substrate. In one embodiment, the step of providing the sample and a plurality of reporter particles having ω different colours to a substrate comprises providing the sample and the plurality of reporter particles having ω different colours simultaneously to the substrate, e.g. as a mixture. In one embodiment therefore, the method comprises incubating the sample with the plurality of reporter particles having ω different colours to form a mixture and providing the mixture to the substrate. In one embodiment, the plurality of reporter particles having ω different colours are pre-disposed in the substrate. When the method is implemented in a paper or membrane-based diagnostic platform such as LFA, the reporter particles may be pre-disposed in a region of the lateral flow substrate, for example, in the conjugate pad. The reporter particles may be dried down and held in place in the conjugate pad. In some embodiments, wherein the reporter particles are pre-disposed in the substrate, the substrate is configured to release the reporter particles on contact with the sample.

In one embodiment, the method further comprises washing the substrate after the providing the sample and the plurality of reporter particles. In some embodiments, washing the substrate comprises washing the substrate with buffers such as phosphate buffered saline (PBS) and bovine serum albumin (BSA). Advantageously, the washing step may remove non-specific binding of target analytes and reporter particles and/or may remove target analytes and reporter particles that are not substantially immobilized onto the substrate.

In one embodiment, the reporter particles of each of ω different colours is configured to couple to at least two different target analytes. In various embodiments, the two different target analytes are different genuses/categories/types of target analytes (e.g. protein/polypeptides vs nucleic acids/polynucleotides). For example, reporter particles of cyan colour may be configured to couple to a polypeptide-based target analyte and a polynucleotide-based target analyte which are immbolised on different assigned spatial regions on the substrate. As may be appreciated, this increases the multiplexing capability of the method without increasing the number of inputs or parameters for detection.

In various embodiments, the reporter particles of each of ω different colours comprise nanostructures. In one embodiment, the nanostructures are zero-dimensional. In one embodiment, the reporter particles of each of ω different colours comprise nanoparticles. In various embodiments, zero-dimensional nanoparticles have more planes of symmetry than one-dimensional nanorods and/or two-dimensional nanoplates. Without being bound by any theory, it is believed that the symmetry of zero-dimensional nanoparticles result in the nanoparticles having a single plasmonic absorption, which could benefit analysis based on colorimetry involving colocalization of two or more colours in the same spatial region as described herein.

Accordingly, in the one embodiment, the reporter particles of each of ω different colours have a single localized surface plasmon resonance (LSPR) absorption peak. In various embodiments, the reporter particles of each of ω different colours have a localized surface plasmon resonance (LSPR) absorption peak within the range of from about 550 nm to about 570 nm, from about 555 nm to about 565 nm, from about 560 nm to about 565 nm, about 563 nm, from about 400 nm to about 420 nm, from about 405 nm to about 415 nm, from about 405 nm to about 410 nm, about 407 nm, from about 610 nm to about 630 nm, from about 615 nm to about 625 nm, from about 615 nm to about 620 nm, about 617 nm. In one embodiment, the reporter particles of each of ω different colours comprise or consist of nanoparticles each having a LSPR absorption peak of about 563 nm, about 407 nm or about 617 nm. Advantageously, when the LSPR absorption peak of the reporter particles of each of ω different colours are in accordance with the values disclosed herein, the colour arising from thereporter particles of each of ω different colours, and any secondary colours resulting from the colocalization of reporter particles of two or more different colours from the ω different colours may be visually distinct to a human eye.

In various embodiments, the nanoparticles comprise metal nanoparticles. In some embodiments, the nanoparticles comprise noble metal nanoparticles. In some embodiments, the noble metal nanoparticles comprises nanoparticles of one or more of the following noble metals: gold, silver, platinum, palladium, ruthenium and rhenium. In some embodiments, the reporter particles comprise one or more of the following selected from the group consisting of: gold nanoparticles, silver nanoparticles, gold-silver nanocomposites and any combinations thereof. In further embodiments, the reporter particles comprise one or more of the following selected from the group consisting of: solid gold nanoparticles, solid silver nanoparticles with gold core and hollow gold nanoshells. In one embodiment, the solid gold nanoparticles have a LSPR absorption peak of about 407 nm. In one embodiment, the solid silver nanoparticles with gold core have a LSPR absorption peak of about 563 nm. In one embodiment, the hollow gold nanoshells have a LSPR absorption peak of about 617 nm. As may be appreciated, the composition of the coloured reporter particle may contribute to its property, for example, its LSPR absorption peak.

In one embodiment, the reporter particles comprises colloidal nanoparticles. As may be appreciated, the use of colloidal nanoparticles are advantageous due to their convenient colorimetric readout and single-step operation, which may greatly enhance the user experience, and significantly reduce the assay cost and turnaround time, as compared to the use of other types of reporter particles/conjugates such as fluorescent molecules, quantum dots, upconverting phosphor and enzymes.

In various embodiments, the reporter particles exhibit one or more of the following morphologies: truncated octahedral structure, truncated cubic structure and hollow cubic structure. As may be appreciated, the morphology of the coloured reporter particle may contribute to its property, for example, its LSPR absorption peak.

In various embodiments, the reporter particle comprise a detecting agent coupled to a nanoparticle, the detecting agent configured to preferentially couple, for example by way of a label, or bind to a target analyte or portions/fragments thereof. In various embodiments, the detecting agent is selected from the group consisting of: polynucleotides, oligonucleotides, aptamers, molecular probes, antibodies, proteins, peptides, organic compounds, small molecules, compounds and molecules capable of interacting with the analyte or portions/fragments thereof. In one embodiment, the reporter particle comprises a nanoparticle-antibody conjugate.

In various embodiments, the method further comprises preparing the reporter particles. In various embodiments, preparing the reporter particles comprises adding a strong reducing agent to a metal ion or salt solution, for example adding a sodium borohydride (NaBH₄) or the like to Au³⁺ solution (Au(III) solution) in the presence of cetyltrimethylammonium bromide (CTAB) and/or cetyltrimethylammonium chloride (CTAC) and/or citrate to form Au seed particles. In some embodiments, preparing the reporter particles further comprises adding a growth mixture to the metal seed particles to form the nanoparticles. The growth mixture may comprise a weak acid, an alkali, a detergent, a weak reducing agent and/or a metal ion/salt solution. For example, the method may comprise adding CTAB, Au³⁺ and a weak reducing agent such as ascorbic acid or the like to the Au seed particles to form the Au nanoparticles or the solid gold nanoparticles. Accordingly, in various embodiments, the growth mixture and/or metal seed particles are precusors of the reporter particles e.g. nanoparticles. In some embodiments, preparing the reporter particles further comprises adding a weak reducing agent such as ascorbic acid, sodium hydroxide (NaOH), the Au seed particles and Ag⁺ to CTAB/CTAC/citrate to form the Au@Ag nanoparticles or the solid silver nanoparticles with gold core. In further embodiments, the weak reducing agent such as ascorbic acid, the NaOH, the Au seed particles and the Ag⁺ are added sequentially to CTAB/CTAC/citrate to form the Au@Ag nanoparticles or the solid silver nanoparticles with gold core. In some embodiments, preparing the reporter particles further comprises adding a gold salt solution, preferably a gold(III) salt solution such as chlorauric acid (HAuCl₄), potassium tetrabromoaurate(III) (KAuBr₄), gold(III) chloride (AuCl₃) or sodium tetrachloroaurate(III) (NaAuCl₄) to the Au@Ag nanoparticles in solution to form the Au/Ag hollow nanoparticles or the hollow gold nanoshells. In further embodiments, the gold salt solution is added dropwise to the Au@Ag nanoparticles in solution to form the Au/Ag hollow nanoparticles or the hollow gold nanoshells. In yet further embodiments, the gold salt solution is added dropwise to the Au@Ag nanoparticles in solution until a cyan solution is obtained. In one embodiment, the gold salt solution comprises HAuCL₄. As may be appreciated, by using a seed mediated growth method, the size and shape of resulting nanostructures may be better controlled, thereby avoiding flutations of the nanoparticles during the growth stage, leading to the formation of exclusively single-crystalline nanoparticles. In one embodiment therefore, the nanoparticles comprises single-crystalline nanoparticles.

In various embodiments, the substrate comprises a capturing agent disposed thereon configured to immobilise a target analyte thereto. In various embodiments, each assigned spatial region on the substrate comprises a capturing agent that is different from capturing agents disposed on the other assigned spatial regions. Accordingly, in various embodiments, each assigned spatial region on the substrate comprises a capturing agent that is configured to immobilise a target analyte that is different from those immobilised by the capturing agents disposed on the other assigned spatial regions. In various embodiments, the capturing agent is selected from the group consisting of: polynucleotides, oligonucleotides, aptamers, molecular probes, antibodies, proteins, peptides, organic compounds, small molecules, compounds and molecules capable of interacting with the analyte or portions/fragments thereof. In one embodiment, the capturing agent comprises a streptavidin. As may be appreciated, streptavidin has very high binding affinity for biotin. Accordingly, in some embodiments, the capturing agent is configured to immobilise the target analyte by binding to the label of the target analyte. When the method is implemented in a paper or membrane-based diagnostic platform such as LFA, the capturing agent may be disposed in a region of the lateral flow substrate, for example, at the designated test regions or lines.

In a particular embodiment, the capturing agent is selected from the group consisting of: anti-human chorionic gonadotropin (anti-HCG), anti-alpha fetoprotein (anti-AFP) and streptavidin, and the detecting agent comprised in the reporter particles is selected from the group consisting of: anti-HCG, anti-AFP, anti-DIG, anti-FITC and anti-DPN. In a particular embodiment, the capturing agent is selected from the group consisting of: anti-DIG, anti-FITC and anti-DPN, and the detecting agent comprised in the reporter particles is selected from the group consisting of: anti-DIG, anti-FITC and anti-DPN. In one embodiment therefore, the capturing agent is the same as the detecting agent. As may be appreciated, depending on the analyte of interest, other capturing agents may also be used as long as the capturing agent is capable of coupling to target analyte. Other detecting agents may also be used as long as the detecting agent is capable of coupling to target analyte and the nanoparticles of the reporter particles.

In one embodiment, the substrate comprises a lateral flow substrate. In a further embodiment, the substrate comprises a lateral flow strip. Accordingly, in one embodiment, the method comprises a lateral flow based method.

In various embodiments, the substrate comprises a control region/zone/line, the control region/zone/line being configured to immobilise a control sample or the reporter particles. The control region/zone/line may be located distal to the test region/zone/line to prevent colocalization of reporter particles intended for the control region/zone/line, and reporter particles intended for the test region/zone/line. In a particular embodiment, the control region/zone/line comprises goat anti-rabbit antibodies for immobilising the reporter particles.

Accordingly, in some embodiments, the method further comprises contacting the substrate with a control sample for obtaining an indication on whether the method is correctly performed. The control sample may comprise a positive control sample, a negative control sample, or both. As may be appreciated, an immobilization of a positive control sample may indicate that the method is correctly performed, for example, an amplification reaction is successful and that the result obtained is not a false negative due to unsuccessful amplification. No detectable immobilization of a negative control sample may indicate that the method is correctly performed, for example, there is no non-specific interaction and/or non-specific amplification and that the result obtained is not a false positive. In various embodiments, the control sample is coupled to the reporter particles for detection. In some embodiments, the method comprises processing the control sample prior to contacting it with the substrate. As may be appreciated, methods of processing the sample, as herein disclosed before, may apply to the control sample.

In various embodiments, the determining step further comprises comparing the colour in each of the α assigned spatial regions of the substrate with a reference list/table to identify the analytes that are present in the sample.

In various embodiments, each of the β different colours and any combinations of two or more of the β different colours thereof is assigned a unique colour code. In various embodiments therefore, the method further comprises determining the colour in each of the α assigned spatial regions and assigning the corresponding colour code to each region. In one embodiment, wherein there is no detectable colour at a region, a code, that is different from the colour code of each of the β different colours and any combinations of two or more of the β different colours, is assigned to the region. In an embodiment, the colour code comprises a one-letter code. In one embodiment therefore, the method further comprises combining the corresponding colour code/code at each region such that a resulting α-letter code is obtained for determining the presence of different target analytes in the sample. In one embodiment, detecting the presence of different target analytes in the sample comprises matching the α-letter code with a reference list/table of predetermined codes to determine the identity of the different target analytes present in the sample.

In one embodiment therefore, the method further comprises assigning a code to each spatial region based on the colour at the spatial region; and combining the code assigned to each spatial region to obtain a combined code, wherein the combined code is used to determine the presence of different target analytes in the sample. In a further embodiment, the method further comprises comparing the combined code with a list of predetermined codes to determine the presence of different target analytes in the sample. In one embodiment therefore, the method further comprises assigning a code to each spatial region based on the colour at the spatial region; combining the code assigned to each spatial region to obtain a combined code, wherein the combined code is used to determine the presence of different target analytes in the sample; and optionally comparing the combined code with a list of predetermined codes to determine the presence of different target analytes in the sample.

In various embodiments, the method is capable of distinguishing at least two or at least three different subtypes within a first analyte type and at least two or at least three different subtypes within a second analyte type, and wherein the first analyte type is same or different from the second analyte type. For example, embodiments of the method is capable of distinguishing a first analyte type of a DNA from a second analyte type of a protein, and within the first analyte type of a DNA, further distinguishing among subtypes of DNA e.g. a FITC+ DNA-1, a DIG+ DNA-2 and a DNP+ DNA-3 etc, and within the second analyte type of a protein, further distinguishing between subtypes of proteins .e.g an alpha fetoprotein (AFP) and a human chorionic gonadotropin (HCG), all within a single substrate i.e. a single test. In a further example, embodiments of the method is capable of distinguishing among the subtypes herpes simplex virus-1 (HSV-1), HSV-2 and HSV-3 of HSV virus. Embodiments of the method are therefore highly sensitive and highly specific, notwithstanding the high level of multiplexing. Embodiments of the method may therefore produce accurate results, which is particularly important in a clinical and/or a laboratory setting.

In one embodiment, the method is capable of detecting up to at least N analytes, wherein N is more than α, β and/or ω.

In one embodiment, the method comprises a diagnostic method. The method is an in vitro method.

In one embodiment, there is provided a kit for detecting different target analytes in a sample, the kit comprising: a substrate that comprises α assigned spatial regions, each spatial region being configured to immobilize a target analyte that is different from the other assigned spatial regions; and a plurality of reporter particles having ω different colours or precursors thereof, wherein the ω different colours are such that a secondary colour resulting from a combination of two or more of the ω different colours is distinct from other combinations of the ω different colours, wherein the reporter particles of each different colour are configured to couple to a target analyte that is different from that of the reporter particles of the other different colours, and wherein α is an integer that is at least two and ω is an integer that is at least three.

Precursors of the reporter particles may be provided in the kit to allow a user to synthesize the reporter particles. In various embodiments, the precursors comprise one or more of a strong reducing agent (e.g. NaBH₄), CTAB, CTAC, citrate, Au salt, HAuCl₄, KAuBr₄, AuCl₃ NaAuCl₄, Au seed particles, a weak reducing agent (e.g. ascorbic acid), NaOH, Ag salt or AgNO₃. The Au seed particles may refer to Au seed particles obtained by embodiments of the methods disclosed herein.

In various embodiments, the kit further comprises instructions on synthesizing the reporter particles from the precursors. The instructions may include one or more steps of the method disclosed herein for preparing the reporting particles.

In various embodiments, it will be appreciated that the plurality of reporter particles having ω different colours within the kit is not intended to be only limited as a component that is separate from the substrate, as alternatively they may be physically in contact with the substrate. In one embodiment, wherein the plurality of reporter particles having ω different colours are provided separately, the plurality of reporter particles having ω different colours are provided in the form of colloidal particles. In one embodiment, the plurality of reporter particles having ω different colours are predisposed on the substrate.

In one embodiment of the kit, ω is three.

In various embodiments, the reporter particles in the kit comprise one or more features as described hereinabove.

In some embodiments, the kit further comprises an oligonucleotide for hybridizing to at least a portion of a polynucleotide/nucleic acid of the target analyte or its amplicon thereof. In some embodiments, the oligonucleotide is coupled to a label that is configured to in turn couple to the reporter particles of at least one of ω different colours. In one embodiment therefore, the oligonucleotide comprises a labelled oligonucleotide that is configured to couple to reporter particles of at least one of ω different colours.

In one embodiment, the label is capable of interacting with or coupling to reporter particles. In one embodiment, the label is capable of interacting with or coupling to a capturing agent which may be disposed on the substrate. In a preferred embodiment, each label is capable of interacting with or coupling to reporter particles of one of ω different colours or one of the capturing agents which may be disposed on the substrate. In one embodiment, the label comprises a ligand or a hapten. In various embodiments, the ligand or hapten is selected from the group consisting of: biotin, DIG, DNP, fluorescein such as 6-FAM or FITC, Texas red, TAMRA, or the like and any combinations thereof.

In one embodiment, the oligonucleotide comprises a primer. In some embodiments therefore, the kit comprises a primer or a labelled primer. Accordingly, in some embodiments, the kit comprises a first oligonucleotide, e.g. a labelled primer, that is configured to label a first strand of a polynucleotide/nucleic acid of the target analyte or its amplicon thereof, a second oligonucleotide, e.g. a labelled primer, configured to label a complementary strand of a polynucleotide/nucleic acid of the target analyte or its amplicon thereof, or both. In some embodiments, the kit comprises two different oligonucleotides, e.g. two different labelled primers, wherein each oligonucleotide is configured to hybridize to one of the two complementary strands of a double-stranded polynucleotide/nucleic acid of the target analyte or its amplicon thereof to dually label the polynucleotide/nucleic acid of the target analyte or its amplicon thereof. In one embodiment, the kit comprises a first oligonucleotide, e.g. a labelled primer, that is coupled to a ligand or hapten selected from biotin and Texas Red; and a second oligonucleotide, e.g. a labelled primer, that is coupled to a hapten selected from the group consisting of digoxigenin (DIG), dinitrophenol (DNP), fluorescein such as 6-FAM or FITC, and TAMRA.

In various embodiments, the kit comprises at least four primers, at least six primers, at least eight primers, at least ten primers, at least 12 primers, at least 14 primers, at least 16 primers or at least 18 primers. In various embodiments, the kit further comprises one or more of the following selected from the group consisting of: deoxynucleotides (NTPs), magnesium sulfate (MgSO₄), magnesium ion, isothermal buffer, DNA polymerase and isothermal buffer.

In various embodiments, the substrate in the kit comprises one or more features as described hereinabove. In one embodiment, the substrate comprises capturing agents disposed on the α different assigned spatial positions of the substrate for immobilizing the target analytes, wherein the capturing agents in each different spatial region preferentially bind to a different target analyte. In one embodiment, the capturing agent is selected from the group consisting of: polynucleotides, oligonucleotides, aptamers, molecular probes, antibodies, proteins, peptides, organic compounds, small molecules, compounds and molecules capable of interacting with the analyte or portions/fragments thereof. In one embodiment, the capturing agent comprises a streptavidin. In one embodiment, the substrate comprises a lateral flow strip. In one embodiment, the substrate comprises a control region/zone/line, the control region/zone/line being configured to immobilise a control sample or the reporter particles.

Accordingly, in various embodiments, the kit further comprises a control sample that is configured to be immobilised in a control region/zone/line on the substrate to indicate that the method is correctly performed. In some embodiments, the control sample comprises an equivalent, a copy or a clone of the target analyte and/or fragments/subtypes/metabolites thereof. In some embodiments, the kit further comprises control primers for amplifying a polynucleotide or a nucleic acid in the control sample. As may be appreciated, the detection of amplicons of the control sample at the control region or line on the substrate may indicate that the results obtained by the kit is valid.

In various embodiments, the kit further comprises instructions on assigning a code to each spatial region selected from the α spatial regions based on the colour at the spatial region and combining the code assigned to each spatial region to obtain a combined code for determining the presence of different target analytes in the sample. In further embodiments, the kit further comprises a list of predetermined codes for comparison with the combined code to determine the presence of different target analytes in the sample. In some embodiments therefore, the kit further comprises instructions on assigning a code to each spatial region selected from the α distinct regions based on the colour at the spatial region and combining the code assigned to each spatial region to obtain a combined code for determining the presence of different target analytes in the sample; and optionally a list of predetermined codes for comparison with the combined code to determine the presence of different target analytes in the sample.

In various embodiments, the kit is capable of detecting up to at least N analytes, wherein N is more than α, β and/or ω.

In one embodiment, the kit is portable. In one embodiment, the kit is reusable. In one embodiment, the kit is disposable. In one embodiment, the kit comprises a diagnostic kit. In one embodiment, the diagnostic kit comprises a point-of-care diagnostic kit.

### BRIEF DESCRIPTION OF FIGURES

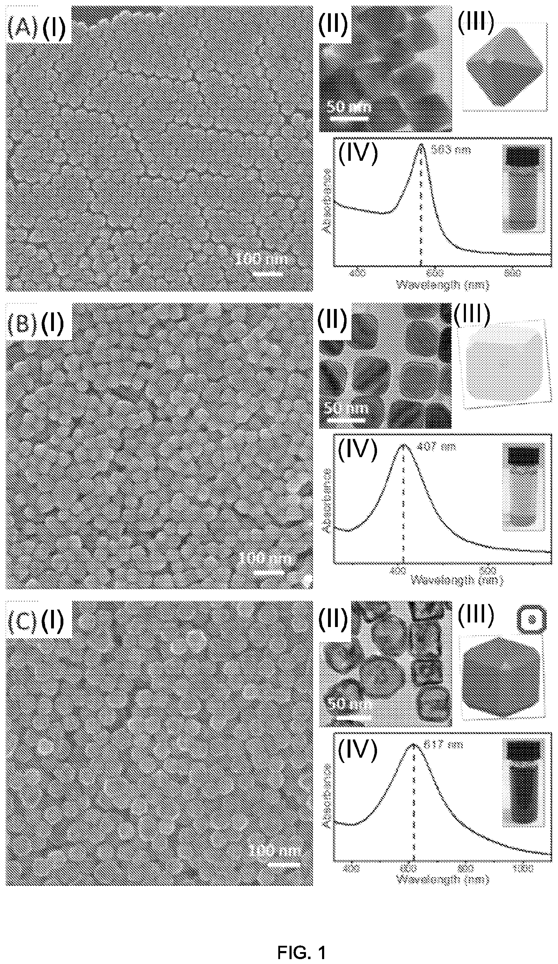

FIG.1 shows the characterization of coloured reporter particles comprising Au/Ag nanoparticles (NPs) in accordance with one embodiment disclosed herein. (A)(I) shows the scanning electron microscope (SEM) image of Au NPs in accordance with one embodiment disclosed herein. (A)(II) shows the transmission electron microscopy (TEM) image of Au NPs in accordance with one embodiment disclosed herein. (A)(III) shows the geometric model of Au NPs in accordance with one embodiment disclosed herein. (A)(IV) shows the UV-vis spectrum of Au NPs in accordance with one embodiment disclosed herein. (B)(I) shows the SEM image of Au@Ag core-shell NPs in accordance with one embodiment disclosed herein. (B)(II) shows the TEM image of Au@Ag core-shell NPs in accordance with one embodiment disclosed herein. (B)(III) shows the geometric model of Au@Ag core-shell NPs in accordance with one embodiment disclosed herein. (B)(IV) shows the UV-vis spectrum of Au@Ag core-shell NPs in accordance with one embodiment disclosed herein. (C)(I) shows the SEM image of Au/Ag hollow NPs in accordance with one embodiment disclosed herein. (C)(II) shows the TEM image of Au/Ag hollow NPs in accordance with one embodiment disclosed herein. (C)(III) shows the geometric model of Au/Ag hollow NPs in accordance with one embodiment disclosed herein. (C)(IV) shows the UV-vis spectrum of Au/Ag hollow NPs in accordance with one embodiment disclosed herein.

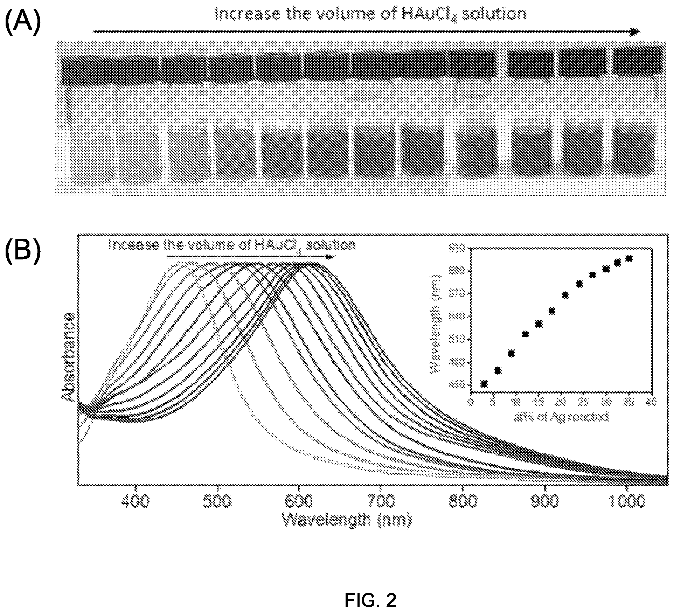

FIG. 2 shows that galvanic replacement of the Au@Ag core-shell NPs with HAuCL₄ solution allowed for tuning of the LSPR peak of the Au/Ag NPs spanning the visible spectrum in accordance with one embodiment disclosed herein. (A) shows that Au/Ag NPs prepared by reacting Au@Ag core-shell NP solution with different volumes of a 20 mM HAuCL₄ solution exhibited different colours in accordance with one embodiment disclosed herein. (B) shows the corresponding UV-visible absorbance spectra of Au/Ag NP solutions shown in (A) in accordance with one embodiment disclosed herein.

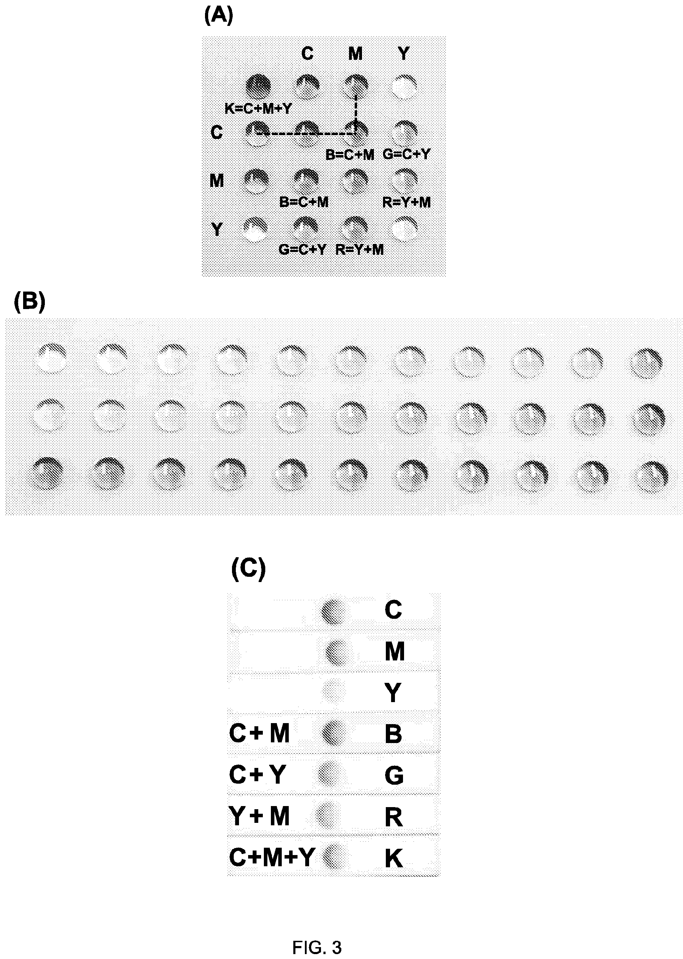

FIG. 3 shows the colour combinations using NPs of three primary colours in accordance with one embodiment disclosed herein. (A) shows that combination of NPs of three primary colours resulted in NP mixtures of unique secondary colours in accordance with one embodiment disclosed herein. (B) shows the colour palette obtained by mixing primary NPs at various ratios in accordance with one embodiment disclosed herein. (C) shows FITC-labeled dsDNA target being detected by single primary coloured NPs and a mixture of NPs in accordance with one embodiment disclosed herein.

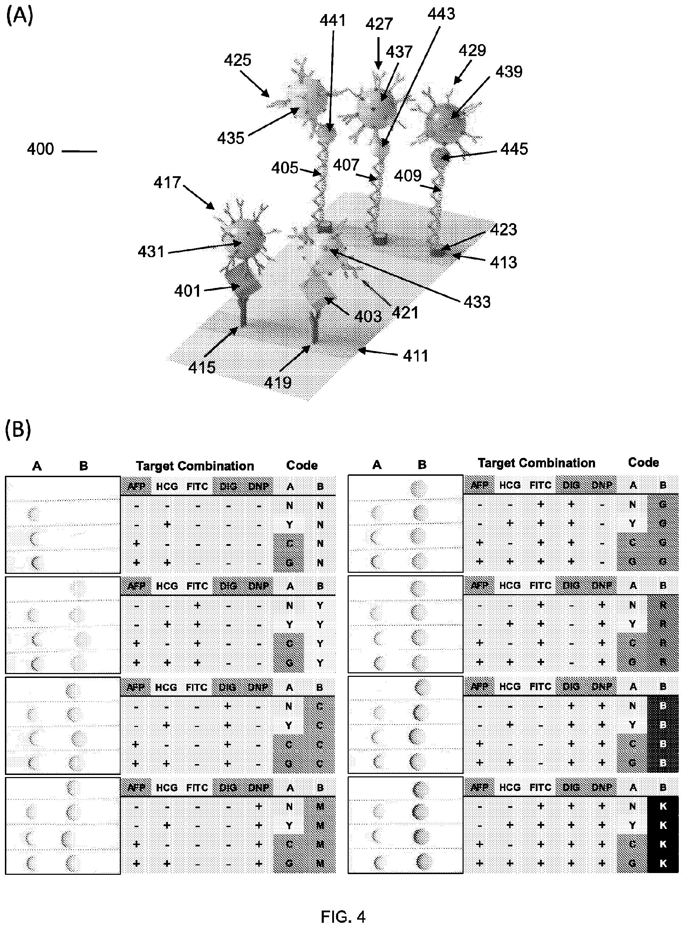

FIG. 4 illustrates two-parameter multiplexing based on spatial separation and colour colocalization in accordance with one embodiment disclosed herein. (A) depicts the detection scheme in accordance with one embodiment disclosed herein. (B) shows the multiplexed detection of all 32 possible target combinations in accordance with one embodiment disclosed herein.

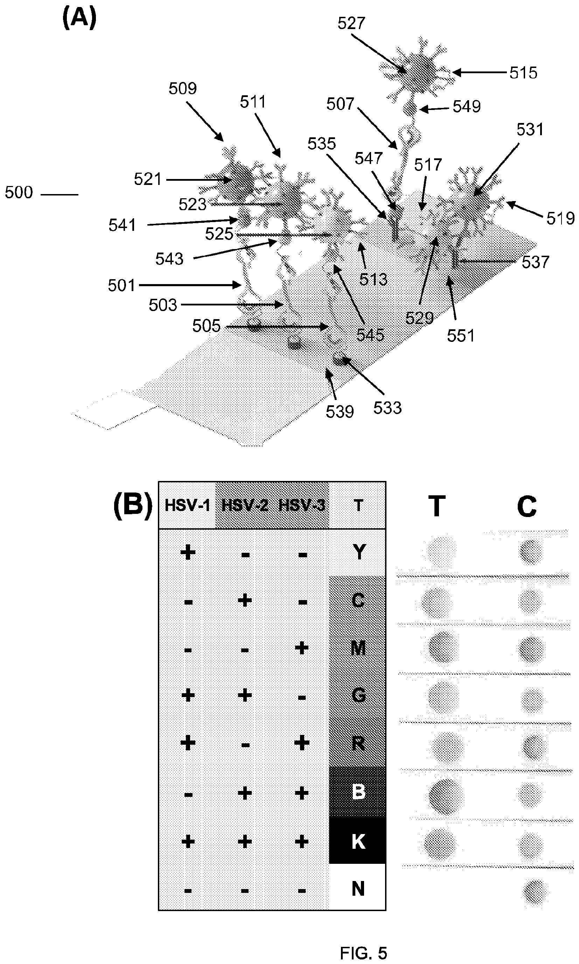

FIG. 5 illustrates multiplexed LFA for HSV subtyping with two-parameter multiplexing strategy in accordance with one embodiment disclosed herein. (A) is a schematic illustration of the multiplex HSV subtyping in accordance with one embodiment disclosed herein. (B) shows the multiplexed detection of all 8 target combinations including a negative sample in accordance with one embodiment disclosed herein.

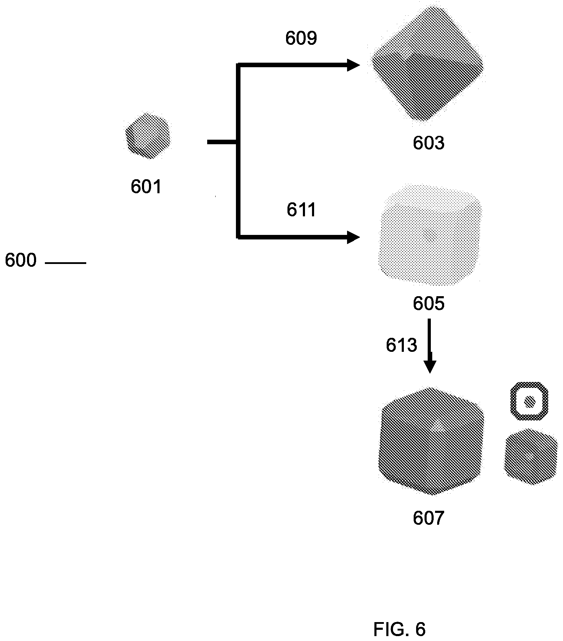

FIG. 6 is a schematic illustration of the preparation of multicoloured Au/Ag NPs i.e. Au NPs (magenta solution), Au@Ag NPs (yellow solution) and Au hollow NPs (cyan solution) in accordance with one embodiment disclosed herein.

### DETAILED DESCRIPTION OF FIGURES

FIG.1 shows the characterization of coloured reporter particles comprising Au/Ag nanoparticles (NPs) in accordance with one embodiment disclosed herein.

FIG. 1(A)(I) and (A)(II) show the general product morphology and the structure of Au NPs respectively in accordance with one embodiment disclosed herein. As shown, the Au NPs exhibited a truncated octahedral shape similar to that illustrated in (A)(III). Further, the Au NPs had an absorption peak at about 563 nm as shown in (A)(IV).

FIG. 1(B)(I) and (B)(II) show the general product morphology and the structure of Au@Ag core-shell NPs respectively in accordance with one embodiment disclosed herein. As shown, the Au@Ag core-shell NPs exhibited a truncated cubic shape similar to that illustrated in (B)(III). Further, the Au NPs had an absorption peak at about 407 nm as shown in (A)(IV).

FIG. 1(C)(I) and (C)(II) show the general product morphology and the structure of Au/Ag hollow NPs respectively in accordance with one embodiment disclosed herein. As shown, the Au/Ag hollow NPs exhibited a hollow cuboid structure similar to that illustrated in (C)(III). Further, the Au/Ag hollow NPs had an absorption peak at about 617 nm as shown in (A)(IV).

FIG. 2 shows that galvanic replacement of the Au@Ag core-shell NPs with HAuCL₄ solution allowed for tuning of the LSPR peak of the Au/Ag NPs spanning the visible spectrum in accordance with one embodiment disclosed herein.

FIG. 2A shows that dropwise addition of 20 mM HAuCL₄ to the Au@Ag core-shell NP solution to obtain Au/Ag NPs solution gradually changed the colour of the solution from yellow to orange, to reddish brown and finally to cyan in accordance with one embodiment disclosed herein. The colours were visually distinguishable.

FIG. 2B shows the corresponding absorbance spectra of the solutions in FIG. 2A with dropwise addition of 20 mM HAuCL₄ to the Au@Ag core-shell NP solution in accordance with one embodiment disclosed herein. As shown, the LSPR peak position of the Au/Ag NPs was tunable from about 453 nm to about 620 nm with HAuCL₄ solution volumes.

FIG 3A shows that combination of NPs of cyan, magenta and yellow (designated by C, M and Y respectively) resulted in NP mixtures of unique secondary colours, such as blue (designated by B) arising from a mixture of cyan and magenta NPs, green (designated by G) arising from a mixture of cyan and yellow NPs, red (designated by R) arising from a mixture of yellow and magenta NPs and black (designated by K) arising from a mixture of all three cyan, magenta and yellow NPs in accordance with one embodiment disclosed herein. These colours were unique and easily distinguishable by unaided eyes.

FIG. 3B shows the colour palette obtained by mixing primary NPs at various ratios in accordance with one embodiment disclosed herein. Across the first row, decreasing concentration of yellow NPs and a corresponding increasing concentration of magenta NPs led to a gradual colour change from yellow to red to magenta. Across the second row, decreasing concentration of yellow NPs and a corresponding increasing concentration of cyan NPs led to a gradual colour change from yellow to green to cyan. Across the third row, decreasing concentration of magenta NPs and a corresponding increasing concentration of cyan NPs led to a gradual colour change from magenta to blue to cyan.

FIG. 3C shows FITC-labeled dsDNA target being detected by the primary colours (cyan, magenta and yellow designated by C, M and Y respectively) of single coloured NPs and the secondary colours (blue, green, red and black designated by B, G, R and K respectively) of a mixture of NPs in accordance with one embodiment disclosed herein.

FIG. 4 illustrates two-parameter multiplexing based on spatial separation and colour colocalization in accordance with one embodiment disclosed herein.

FIG. 4A depicts the detection scheme 400. Two protein-based targets in the form of HCG 401 and AFP 403 were detected at a zone 411 (Zone A), and three DNA-based targets 405, 407 and 409 were detected at another zone 413 (Zone B). For each protein target, a pair of detecting agent and capturing agent in the form of antibodies that would bind to different epitopes of the protein, was used. For protein target HCG 401, a detecting agent in the form of anti-HCG 417 and a capturing agent in the form of anti-HCG 415 were used. For protein target AFP 403, a detecting agent in the form of anti-AFP 421 and a capturing agent in the form of anti-AFP 419 were used. The capturing agents anti-HCG 415 and anti-AFP 419 were immobilised on the substrate at zone 411 while the detecting agent anti-HCG 417 and anti-AFP 421 were each conjugated to yellow nanoparticle 431 and cyan nanoparticle 433 respectively. For each DNA target 405, 407 and 409, one end of the DNA was labelled with a ligand in the form of biotin (not shown), and the other end was labelled with a hapten in the form FITC 441, DIG 443 or DNP 445 respectively for each of the DNA targets 405, 407 and 409. A common capturing agent 423 in the form of streptavidin which is a receptor to the ligand biotin, was immobilised on the substrate at zone 413 for capturing the biotin-labelled DNA targets 405, 407 and 409. The detecting agents in the form of anti-FITC 425, anti-DIG 427 and anti-DNP 429 were each conjugated to cyan nanoparticle 435, yellow nanoparticle 437 and magenta nanoparticle 439 respectively. Each of antibody-conjugated nanoparticles 435, 437 and 439 would thus bind to the FITC 441, DIG 443 or DNP 445 of DNA target 405, 407 and 409 respecitvely.

FIG. 4B shows the multiplexed detection of all 32 possible target combinations in accordance with one embodiment disclosed herein. The presence of a target analyte was reported by both the location and the colour of the signal. Colocalization of multiple primary coloured NPs resulted in unique secondary colours. For example, yellow at zone 411 meant the presence of HCG, magenta at zone 413 meant the presence of DNP-labelled DNA, and green at zone 411 arising from a colocalization of yellow and cyan meant the presence of both HCG and AFP. Final results were reported by a combined code, for example, a two-letter code wherein the first letter indicated the colour signal at zone 411 and the second letter indicated the colour signal at zone 413. In the figure, the one-letter code used to designate each colour signal is as follows: C - cyan, M - magenta, Y - yellow, B - blue, G - green, R - red and K - key (black) and N - no visible/detectable colour (nil).

FIG. 5 illustrates multiplexed LFA for HSV subtyping with two-parameter multiplexing strategy in accordance with one embodiment disclosed herein.

FIG. 5A is a schematic illustration 500 of the multiplex HSV subtyping in accordance with one embodiment disclosed herein. Each of the target analytes 501, 503 and 505 corresponding to HSV-1 viral DNA, HSV-2 viral DNA and HSV-3 viral DNA respectively were dually labelled with a ligand biotin (not shown) and a hapten DPN 541, FITC 543 and DIG 545 respectively by use of labelled primers in a loop-mediated isothermal amplification (LAMP) process. A control sample containing a DNA 507 was also subjected to dual labelling with a hapten in the form of Texas red 547 and another hapten in the form TAMRA 549 by use of labelled primers in the same LAMP process. Detecting agents 511 and 519 in the form of anti-FITC were each coupled to yellow nanoparticle 523 and 531 respectively. The yellow nanoparticle 523 would thus be able to couple to the FITC-labelled HSV-2 viral DNA 503. Detecting agent 513 and 517, in the form of anti-DIG, were each coupled to cyan nanoparticle 525 and 529 respectively. The cyan nanoparticle 525 would thus be able to couple to the DIG-labelled HSV-3 viral DNA 505. A detecting agent in the form of anti-DPN 509 and another detecting agent in the form of an anti-TAMRA 515, were each coupled to magenta nanoparticle 521 and 527 respectively. The magenta nanoparticle 521 would thus be able to couple to the DPN-labelled HSV-1 viral DNA 501. The magenta nanoparticle 527 would thus be able to thus couple to the TAMRA-labelled control DNA 507. A capturing agent in the form of streptavidin 533 which is a receptor to the ligand biotin, was immobilised on the substrate at test zone 539 to capture all three biotin/labelled target analytes 501, 503 and 505 and their amplicons thereof. Another capturing agent in the form of an anti-Texas red 535, was immobilised at control zone 551 to capture the Texas red-labelled control DNA 507 and its amplicons thereof. A further capturing agent 537, in the form of a goat anti-rabbit was also immobilised at the control zone 551 to capture excess coloured reporter particles. The detection of a magenta colour at the control zone 551 indicated the successful amplification of the control DNA, and therefore also indicated the successful amplification of the target analytes. The capturing agent 537, in the form of a goat anti-rabbit at the control zone 551 would bind to excess coloured reporter particles 529 and 531. A valid control was when the colour at the control zone 551 is any secondary colour comprising magenta, in other words, a colour other than yellow, cyan, magenta and green. The colour at the test zone 539 revealed what target were present in a sample. For example, if the sample contained HSV-2 viral DNA, the colour at the test zone 539 was cyan. If the sample contained both HSV-1 and HSV-3, the colour at the test zone 539 was red. If the sample contained all three viral subtypes, the colour at the test zone was black. Using embodiments of the method, each of the three viral subtypes and any combinations thereof were successfully identified.

FIG. 5B shows the multiplexed detection of all 8 target combinations including a negative sample in accordance with one embodiment disclosed herein. The colour of the test zone 539 indicated the type(s) of viral DNA present in the sample. Final result was reported by a code indicating the colour signal at the test zone 539, for example, a one-letter code. In the figure, the one-letter code used to designate each colour signal is as follows: C - cyan, M - magenta, Y - yellow, B - blue, G - green, R - red and K - key (black) and N - no visible/detectable colour (nil).

FIG. 6 is a schematic illustration 600 of the preparation of multi-coloured Au/Ag NPs i.e. Au NPs (magenta solution), Au@Ag NPs (yellow solution) and Au hollow NPs (cyan solution) in accordance with one embodiment disclosed herein.

Briefly, a typical synthesis began with the preparation of Au seed particles 601. The addition of reagent 609 in the form of HAuCL₄, to Au seed particles 601 led to the formation of the magenta Au NPs 603. The addition of reagent 611 in the form AgNO₃ to Au seed particles 601 led to the formation of the yellow Au@Ag NPs 605. The further dropwise addition of reagent 613 in the form of HAuCL₄ to the yellow Au@Ag NPs 605 led to the formation of the cyan Au/Ag hollow NPs 607.

### EXAMPLES

Example embodiments of the disclosure will be better understood and readily apparent to one of ordinary skill in the art from the following examples, tables and if applicable, in conjunction with the figures. Example embodiments are not necessarily mutually exclusive as some may be combined with one or more embodiments to form new exemplary embodiments.

In the examples provided herein, the inventors demonstrated a two-parameter multiplexing strategy for paper or membrane-based diagnostic platforms such as LFA based on the spatial separation of the test zone and the colour of the test spot. In these examples, the platform disclosed herein differentiated targets based on the location of the test zone and the colour of the signal readout. Au/Ag NPs of visually distinguishable colours, which were fine-tuned by simply altering their composition and the morphology, were employed as the reporter. Different combinations of targets would display signals at unique locations with distinctive colours at each location. Compared to single-parameter multiplexing strategy, the two-parameter multiplexing strategy in the examples used herein required fewer test zones and fewer particle types to achieve a high degree of multiplexing, which would greatly improve the multiplexing capability and the applicability of LFA.

### Reporter NPs with Tunable Colours

Au/Ag NP systems were selected as the reporter NPs. One of the most useful features of this system was the distinctive and highly tunable optical properties that originated from localized surface plasmon resonance (LSPR). Tight control over the morphology and composition of the NPs allowed one to fine-tune the LSPR peaks over a broad range of the visible spectrum. Such exceptional tunability endowed the Au/Ag NP system with great promise in colorimetric sensing and labelling applications.

0-D Au, Au@Ag and hollow Au/Ag NPs with distinctive plasmonic absorption were employed in this example. 0-D NPs were selected instead of 1-D nanorods and 2-D nanoplates due to their symmetrical shape. Such symmetry resulted in a single plasmonic absorption, which could benefit the unique colocalization feature in the assay design.

Au NPs and Au@Ag NPs were synthesized using a seed-mediated growth method. Au NPs exhibited a truncated octahedral shape with an absorption peak at ~ 563 nm (FIG. 1A). Au@Ag NPs adopted a truncated cubic shape with an absorption peak at ~ 407 nm (FIG. 1B). Galvanic replacement of the Au@Ag NPs with HAuCL₄ solution allowed for straightforward tuning of the LSPR peak of nanostructures spanning the visible spectrum.

FIG. 2A shows the vials of Au/Ag NP solutions prepared by reacting Au@Ag NPs with different volumes of HAuCL₄ solution (20 mM). The colours of the solution were visually distinguishable. The corresponding absorbance spectra (FIG. 2B) indicated that the LSPR peak position of the Au/Ag NPs was tunable from 453 nm to 620 nm with HAuCL₄ solution volumes. The galvanic replacement reaction also resulted in morphological change from a solid cube to a hollow structure (FIG. 1C).

### NP Colour Palette

The inventors selected NPs of three subtractive primary colours for subsequent experiments. The absorption of each of the yellow (Y) NPs, the magenta (M) NPs and the cyan (C) NPs was centred at 407 nm, 563 nm 617 nm, respectively. The selective combination of three primary colours provided an entire palette with visually distinguishable secondary colours (FIG. 3A). By mixing the magenta and cyan NPs, a blue (B) colour was obtained (which looked purplish on the LFA test strip due to particle aggregation). Mixing yellow and cyan NPs resulted in a green colour. The mixture of yellow and magenta NPs gave a red (R) colour. The combination of all three primary colours led to black (K). The aforementioned colours were unique and easily distinguishable by unaided eyes. By mixing the three primary NPs at various ratios, the inventors were able to obtain an entire spectrum of colours (FIG. 3B).

As a proof of concept, the inventors labeled anti-fluorescein isothiocyanate (FITC) antibody primary NPs and NPs mixtures of secondary colours with a total of seven aforementioned colours (C, M, Y, B, G, R and K). The test strip had streptavidin immobilized at the test zone. The target DNA was dually labeled with biotin at one end and FITC at the other end. As the target flowed through the test strip, double-stranded DNA (dsDNA) molecules would accumulate at the test zone via biotin-streptavidin interaction. The antibody-conjugated NPs bound to the FITC at the test zone through the dsDNA target and present various colours (FIG. 3C).

Detector antibodies could be conjugated to a single type of NPs, in which case the test zone would display one of the primary colours. Alternatively, the detector antibodies could be conjugated to a mixture of NPs, whereby different types of NPs would colocalize at the test zone and display the secondary colours. These colours were all visually distinguishable on the test strip.

### Space-Colour Two-Parameter Multiplexing for LFA

Unlike conventional single-parameter multiplexing, the inventors' strategy used a combination of two parameters to achieve a high degree of multiplexing. The two parameters the inventors employed for multiplexed LFA were space and colour. The inventors analyzed five targets with NPs of three colours at two different locations on the strip. First, two test zones were spatially separated along the flow paths (FIG. 4A). Two protein-based targets (alpha fetoprotein (AFP) and human chorionic gonadotropin (HCG)) were detected at Zone A, and three DNA-based targets were detected at Zone B. For each protein target, a pair of antibodies that would bind to different epitopes of the protein was used for detection. One of the antibodies was immobilized on the test strip as the capture antibody, and the other antibody was conjugated to the reporter NPs. For each DNA target, one end of the DNA was labeled with biotin, and the other end was labeled with a hapten molecule (FITC, digoxigenin (DIG) or dinitrophenol (DNP)). Streptavidin was used as the common capture agent to be immobilized on the test strip. The detector antibody for each target was conjugated to one of the three primary NPs. Anti-HCG was conjugated to yellow NPs; anti-AFP was conjugated to cyan NPs; anti-FITC was conjugated to yellow NPs; anti-DIG was conjugated to cyan NPs; anti-DNP was conjugated to magenta NPs. All five NP-conjugated antibodies were mixed. As the mixture flowed through the test strip, each type of NP would accumulate at the test zone if the corresponding target was present in the sample. In the absence of corresponding target, the NP would flow downstream without any trace at the test zone.

The presence of a target was reported by both the location and the colour of the signal (FIG. 4B and C). Any of the 32 possible combinations of targets (five targets with 2⁵ combinations could be distinguished based on the colour and location of the signal. Yellow at Zone A would mean the presence of HCG, and magenta at Zone B would indicate there was DNP-labeled DNA. If more than one protein targets or DNA targets were present in the sample, NPs of different primary colours would colocalize at one test zone, resulting in a distinctive secondary colour. Each colour indicated a unique combination of targets. For example, the presence of FITC-labeled and DNP-labeled DNA targets would give rise to a red spot at Zone B, and the presence of HCG-labeled and AFP-labeled protein targets would produce a green spot at Zone A. Final results were reported by a two-letter colour code where the first letter and second letter would indicate the colour signals at Zone A and Zone B, respectively (FIG. 4B). For instance, CB was translated to AFP+, HCG-, FITC-, DIG+ and DNP+. GK indicated the presence of all five targets. NR (N for Nil) implied the absence of either protein target, and only FITC and DNP-labeled dsDNA targets were present. Using the two-parameter multiplexing strategy, the inventors successfully analyzed all 32 possible combinations of the targets (FIG. 4B).

A unique feature of platform presently disclosed was the two-parameter strategy the inventors employed to facilitate a high-degree of multiplexing. One parameter was the spatial separation. Targets were grouped and spatially separated on the test strip. The location of the signal would indicate which group the target belonged to. The other parameter relied on the colour. When NPs of different primary colours colocalized at the same test zone, it would lead to a distinctive secondary colour that contained the target information. The two-parameter multiplexing has significant advantages over the single-parameter approach. Given the same number of input N for each parameter, a single-parameter multiplexing can only detect N targets with 2^{N} number of different combinations. In a two-parameter multiplexing, N input for each parameter allows the detection of 2N number of targets with 2^{2N} number of different combinations. Therefore, the two-parameter strategy considerably improves the multiplexing capability. The two-parameter strategy could also reduce the number of required input, but still achieve the same degree of multiplexing.

Take the experiment in FIG. 4 for instance, to detect five targets, the traditional single-parameter multiplexing platform requires five separate test zones. In the context of manufacturing, most liquid printing equipment has a limited number of printer head, which means the five test zones have to be printed in multiple runs. In each run, the printer heads would need to be washed and the antibody solutions would have to be reloaded, which would result in waste of reagents and significant time delay. In contrast, the two-parameter strategy would only require two test zones, which could be easily printed in a single run. This would greatly reduce the cost and manufacturing cycle. In the colour-based single-parameter multiplexing, it is difficult to go beyond three targets due to spectral overlap. The NPs colocalization would result in colours indistinguishable from the base colours. For example, if one uses green particles, in addition to the three primary colours, to detect the fourth target, the colocalization of cyan and green would yield a dark greenish colour that is implausible to distinguish by unaided eyes.

### Multiplexed Subtyping of Herpes Virus

Herpes virus is a large family of DNA viruses that causes various types of disease in human. Some of the widely spread herpes viruses include herpes simplex virus-1 (HSV-1) and HSV-2, which cause oral and genital herpes, and varicella zoster virus (also known as HSV-3), which causes chicken pox and shingles. In this example, the inventors applied the two-parameter multiplexing strategy to detect the aforementioned three herpes viruses by amplifying the viral DNA using loop-mediated isothermal amplification (LAMP), and detecting LAMP amplicons using multiplexed LFA. Three target-specific LAMP primer sets and one internal control primer set were used in each reaction (Table 1 below).

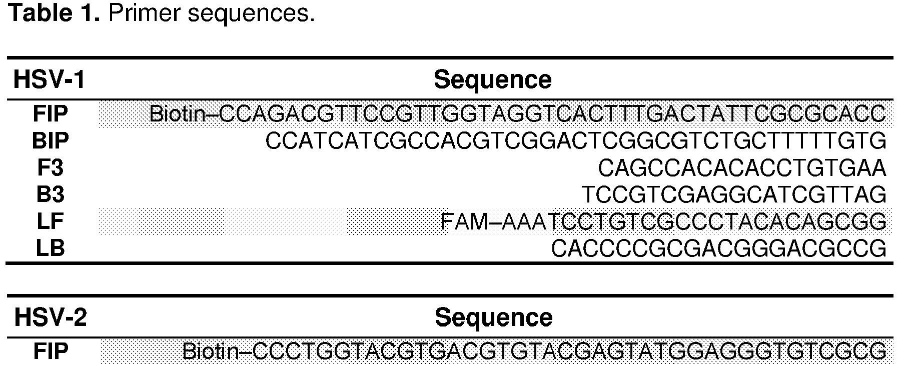

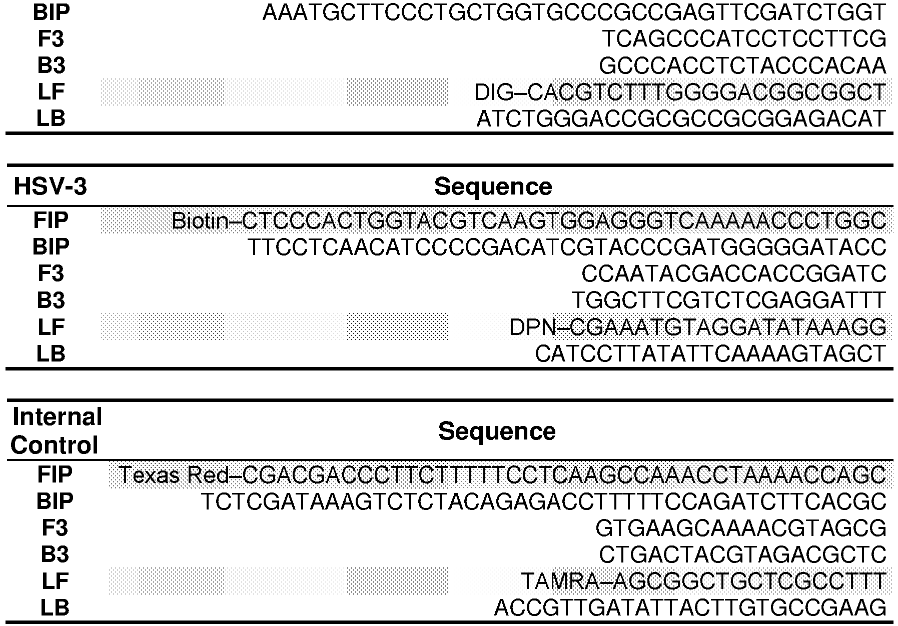

Each primer set contained six primers. In HSV-1, HSV-2 and HSV-3 primer sets, the FIP primers were labeled with biotin, and the LF primers were labeled with FITC, DIG and DPN, respectively. In the internal control primer set, the FIP primer was labeled with Texas red, and the LF primer was labeled with 5-carboxytetramethylrhodamine (TAMRA). Anti-FITC, anti-DIG antibodies were labeled with yellow and cyan NPs respectively, and anti-DPN and anti-TAMRA antibodies were labeled with magenta NPs. Streptavidin was immobilized at the test zone to capture all three target amplicons. Anti-Texas red antibody was immobilized at the control zone to capture the internal control amplicon. Goat-anti-rabbit antibody was also immobilized at the control zone to capture excess anti-FITC yellow NPs and anti-DIG cyan NPs (FIG. 5A).

In the presence of the target, the viral DNA was amplified to a detectable level, and each amplicon was dually labeled with biotin and a hapten molecule. As the amplicon flowed through the LFA test strip, it was captured at the test zone via biotin-streptavidin interaction. The antibody-conjugated NP would recognize the hapten and tag the amplicon with the corresponding NP to reveal its identity (FIG. 5A). The internal control ensured successful LAMP reaction. The internal control amplicon would be tagged by the magenta NP at the control zone. The goat-anti-rabbit antibody at the control zone would bind to excess yellow and cyan NPs. In the end, the colour of the test spot revealed what targets were present in the sample (FIG. 5B). For example, if the sample contained HSV-2, the test spot was cyan in colour. If the sample contained both HSV-1 and HSV-3, the test spot was red in colour. If the sample contained all 3 types of viruses, the spot appeared black.

The inventors successfully identified all three viruses and their combinations in the sample using this method. Ideally, the control spot should be black in colour. However, due to the fact that different amount of cyan and yellow NPs were consumed at the test spot, the three primary NPs might be present at different ratios at the control spot. Therefore, as long as the control spot comprised of at least two primary colours and one of which was magenta, it was considered a valid control. In other words, a valid control spot could be any colour other than yellow, cyan, magenta or green.

In summary, the inventors developed a two-parameter multiplexing strategy for paper or membrane-based diagnostic platforms such as LFA based on the spatial separation of the test zone and the colour of the test spot. First, different groups of targets were separated to distinct test zones on the LFA test strip. In addition, the inventors selected morphology and composition-controlled Au/Ag NPs of three primary colours as the reporter particles. When conjugated to antibody, each type of primary NP would report the presence of the corresponding target by its colour. The colocalization of the primary NPs would produce an array of secondary colours that indicated various target combinations. Compared to traditional single-parameter multiplexing, the two-parameter strategy would greatly improve the multiplexing capability of LFA. Using the two-parameter multiplexing strategy, the inventors successfully distinguished all possible combinations of two protein targets and three DNA targets using LFA. The inventors also successfully performed the multiplexed subtyping of three herpes viruses by combining LAMP and LFA.

### Synthesis of Au/Ag NPs

The overall synthesis route of NPs with three primary colours is illustrated in FIG. 6.

A typical synthesis began with the preparation of Au seed NPs for the synthesis of Au NPs and Au@Ag core-shell NPs. Seed-mediated growth method provided better control over the size and shape of metal nanostructures. Adoption of single-crystalline seeds with relatively large size in seed-mediated growth method avoided the structure fluctuations of the NPs during the growth stage, and led to the formation of exclusively single-crystalline NPs.

The synthesis of Au seed NPs was based on seed-mediated growth with NaBH₄-reduced Au seeds. For the preparation of NaBH₄-reduced Au seeds, 7 mL of 75 mM cetyltrimethylammonium bromide (CTAB) solution was first prepared by dissolving CTAB at 30 ºC with constant stirring. 87.5 µL of 20 mM HAuCL₄ solution was added to the CTAB solution. 0.6 mL of an ice-cold NaBH₄ solution (10 mM) was then injected quickly into the mixture under vigorous mixing to form a brown seed solution. Stirring continued gently at 30 °C for 2-5 h to decompose the excess NaBH₄. A growth solution was prepared by adding 90 µL of 20 mM HAuCL₄ solution and 1.392 mL of 38.8 mM ascorbic acid (in the stated order) into 43 mL of 16.7 mM CTAB solution in a clean test tube at 30 ºC with thorough mixing after each addition. 0.54 mL of the NaBH₄-reduced seed solution was added to the growth solution and thoroughly mixed. The mixture was left unperturbed at 30 ºC overnight.

For the preparation of Au NPs (magenta solution), 0.36 mL of the Au seed NP solution was added to 45 mL of a growth mixture containing 16 mM CTAB, 0.04 mM HAuCI4 and 1.2 mM ascorbic acid. The mixture was thoroughly mixed and left unperturbed overnight. The colour of the solution changed to magenta, indicating the formation of Au NPs. The Au NPs adopted a truncated octahedral morphology with good monodispersity in both size and shape (FIG. 1A). The rhombic projections with slight truncations in the corners in the TEM image are the NPs viewed in <110> directions.

For the preparation of Au@Ag core-shell NPs (yellow solution), 4.64 mL of 38.8 mM ascorbic acid, 180 µL of 1 M NaOH, and 1.8 mL of Au seed solution were added to 38 mL 18.9 mM CTAB solution in the stated order, followed by addition of 0.45 mL of 20 mM AgNO3. The mixture was thoroughly mixed and left on a thermoshaker at 40 ºC overnight. The colour of the solution changed to yellow, indicating the formation of Ag shells. The Au@Ag NPs adopted a truncated cubic morphology (FIG.1B). The Au cores can be clearly observed from the TEM image with a darker contrast.

For the preparation of Au/Ag Hollow NPs (cyan solution), 157.4 µL of 20 mM HAuCL₄ aqueous solution was added dropwise to 45 mL of as-synthesized Au@Ag core-shell NP solution on a thermoshaker. Ag shell would be oxidized by HAuCL₄ according to 3Ag + AuCl₄- → Au + 3Ag+ + 4CI-, because the standard reduction potential of AuCl₄-/Au (1.002 V vs. standard hydrogen electrode (SHE)) was more positive than that of Ag+/Ag (0.7996 V vs SHE). The resulted Au atoms would deposit on the Ag shell template and adopt its morphology, as interior Ag was oxidized to produce a hollow structure. According to reaction stoichiometry, three Ag atoms were removed from the Ag shell with the deposition of one Au atom. The amount of Au would be 33.3 at% of Ag to fully replace the entire Ag shell, and to produce the hollow Au/Ag nanostructures subsequently. The hollow interior was clearly visible in the TEM image (FIG. 1C). The solution gradually changed from yellow to orange, to reddish brown, and finally to a cyan colour with the dropwise addition of HAuCL₄ solution. HAuCl₄/Ag ratios of 0.03, 0.06, 0.09, 0.12, 0.15, 0.18, 0.21, 0.24, 0.27, 0.3, 0.325 and 0.35 were obtained with the addition of 13.5, 27, 40.5, 54, 67.5, 81, 94.5, 108, 121, 135, 146.2 and 157.4 µL of 20 mM HAuCL₄ to 45 mL of as-synthesized Au@Ag core-shell NP solution. The resulting solutions and the corresponding UV-vis spectra are shown in FIG. 2A and 2B.

### Materials Characterizations

The structure of the NPs was analyzed by transmission electron microscopy (TEM) on FEI Tecnai F20 electron microscope operating at the 200 kV accelerating voltage. Field emission scanning electron microscopy (FESEM) (JEOL JSM-7400F) was used to examine the general product morphology. TEM samples were prepared by dispensing a drop of the washed product on a copper grid, followed by drying in air at room temperature. The optical absorption spectra were recorded by a Shimadzu UV-vis-NIR spectrophotometer UV-3600.

### Conjugation of Antibody to Au/Ag NPs

NPs of three subtractive primary colours (cyan, magenta and yellow) were selected for multiplexing in this study. After the synthesis, the NPs were washed by centrifugation at 8,000g for 10 min. The supernatant was then discarded, and the NPs were resuspended in 5 mM phosphate buffer (pH = 5.5). Next, the NPs were subjected to another round of washing under the same condition. Finally, the yellow NPs, magenta NPs and cyan NPs were resuspended in 5 mM phosphate buffer (pH = 5.5) at optical densities (ODs) of 20, 10 and 10, respectively.

The detector antibodies were conjugated to NPs by passive adsorption. A titration was performed to estimate the amount of protein required for labelling. The optimal amount of protein was determined by observing the degree of aggregation. Ideally, the protein would not cause the NPs to aggregate and change colour. It was determined that 5 µg, 2.5 µg and 0.5 µg would be used to conjugate to 1000 OD·µL of cyan, magenta and yellow NPs, respectively.

To conjugate antibodies to NPs, desired amount of antibodies (10 µg for cyan NPs, 5 µg for magenta NPs, and 2.5 µg for yellow NPs) were diluted to 200 µL in 5 mM phosphate buffer (pH = 5.5). 200 µL of NPs at stock concentration (OD = 10 for cyan NPs, OD = 10 for magenta, and OD = 20 for yellow NPs) were mixed with the diluted antibodies and incubated at room temperature for ~ 6 h. After incubation, 200 µL of 10 mM CTAB was added to the mixture and incubated for 10 min. Next, the NPs with antibodies adsorbed on the surface were washed by centrifugation at 2000g for 15 min. The supernatant was discarded, and the particles were resuspended in 200 µL of 5 mM phosphate buffer (pH = 5.5).

For the LFA experiment illustrated in FIG. 3, all NPs were conjugated to anti-FITC (Abcam, Cambridge, United Kingdom). For the LFA experiment illustrated in FIGs. 4 and 5, anti-FITC and anti-HCG (Arista Biologicals, Pennsylvania, USA) were conjugated to yellow NPs. Anti-DIG (Fitzgerald, Massachusetts, USA) and anti-AFP (Thermo Pierce, Illinois, USA) were conjugated to cyan NPs. Anti-DNP (LifeSpan Biosciences, Washington, USA) was conjugated to magenta NPs. For the experiment illustrated in FIG. 5, anti-TAMRA was conjugated to magenta NPs.

### Preparation of LFA Test Strips

For the LFA experiment illustrated in FIG. 3, streptavidin (Promega, Wisconsin, USA) was diluted to 1 mg/mL with 200 mM phosphate buffer (pH = 8), and 1 µL of the diluted streptavidin was dispensed onto the HF135 nitrocellulose test strip (Merck Millipore, Massachusetts, USA). For the LFA experiment illustrated in FIG. 4, 1 µL of streptavidin of the same condition was dispensed onto the test zone B. Capture antibodies of HCG (Arista Biologicals, Pennsylvania, USA) and AFP (Arista Biologicals, Pennsylvania, USA) were mixed and diluted to 0.5 mg/mL each in 200 mM phosphate buffer (pH = 8). 1 µL of the antibody mixture was dispensed onto test zone A. After the dispensing, the strips were dried under vacuum for 30 min. They were then blocked with commercial blocking solution (Candor Bioscience, Wangen, Germany) for another 30 min. Subsequently, the test strips were washed with 5 mM phosphate buffer (pH = 7-7.5 for 1 h. In the end, the strips were dried under vacuum for 3 h before use.

For HSV subtyping, streptavidin (Promega, Wisconsin, USA) was diluted to 1 mg/mL with 200 mM phosphate buffer (pH = 8), and 1 µL of the diluted streptavidin was dispensed onto the test zone of strip (HF135, Merck Millipore, Massachusetts, USA). Anti-Texas red and goat-anti-rabbit was mixed and diluted to 0.5 mg/mL each in 200 mM phosphate buffer (pH = 8). 1 µL of the antibody mixture was dispensed onto the control zone. The rest of the procedure was the same as described above.

### Sample and Assay Conditions

For the LFA experiment illustrated in FIG. 3, only one type of dsDNA was used. An arbitrary 76-bp DNA oligonucleotide T20 (Integrated DNA technology, Iowa, USA) was labeled with biotin at its 5' terminal. Its complementary strand (Integrated DNA technology, Iowa, USA) was labeled with fluorescein at its 5' terminal. The two strands were mixed at equal-molar ratio in 1 × tris buffered saline (1 × TBS, First base technology, Singapore). The mixture was heated at 95 ºC for 10 min, and then allowed to cool down to room temperature. To prepare the sample, the hybridized dsDNA was diluted to 1 µM with 1 × TBS. 5 µL of biotin-FITC dsDNA target was added to 50 µL 1 × phosphate buffered saline (1 × PBS, First base technology, Singapore) supplemented with 5% bovine serum albumin (BSA). The sample was then applied to the test strip. The NP conjugates were added to 50 µL of 1 × PBS supplemented with 5% BSA, and applied to the test strip after the sample had flowed through. For the three primary colours, cyan NPs (2.5 µL), magenta NPs (5 µL), and yellow NPs (10 µL) were used in the respective reaction. For the secondary colours, different NPs of the same volumes mentioned above were mixed in 1 × PBS supplemented with 5% BSA, and applied to the test strip. In the end, the test strip was washed with 50 µL of 1 × PBS supplemented with 5% BSA.

For the LFA experiment illustrated in FIG. 4, a total of five targets were used. Recombinant HCG (Arista Biologicals, Pennsylvania, USA) and AFP (Arista Biologicals, Pennsylvania, USA) antigens were purchased from commercial source. The same 76-bp DNA oligonucleotide T20 was used. Three complementary strands were labeled with FITC, DIG and DNP, respectively. Three dsDNA targets were prepared by hybridizing the biotinylated T20 with the three labeled complementary oligonucleotides. The resulting dsDNA targets were dually labeled with biotin-FITC, biotin-DIG and biotin-DNP, respectively. To prepare the sample, 5 µL of HCG antigen at 10 µg/mL, 5 µL of AFP antigen at 10 µg/mL, and 2 µL of each dsDNA target at 1 µM were added to 50 µL of 1 × PBS supplemented with 5% BSA. Each sample might or might not contain all the targets. The sample was first applied to the test strip, and allowed to flow through. The detector mixture was prepared by mixing the five NP-conjugated detector antibodies (2.5 µL of cyan anti-DIG, 2.5 µL of cyan anti-AFP, 5 µL of magenta anti-DNP, 10 µL of yellow anti-FITC, and 10 µL of yellow anti-HCG) with 25 µL of 1 × PBS supplemented with 10% BSA. The mixture of reporter NPs was applied to the test strip after the sample has flowed through. Lastly, the test strip was washed with 50 µL of 1 × PBS supplemented with 5% BSA.

### HSV Subtypinq

All three types of viral DNA were acquired from American Type Culture Collection (ATCC, Virginia, USA). All primers were ordered from Integrated DNA Technology (IDT DNA, Iowa, USA). The three target-specific primer sets were obtained from Kaneko et al.,41 and the internal control primer set was designed in house using Primer Explorer Software (https://primerexplorer.jp/e/). The LAMP recipe is shown in Table 2 below. The LAMP reagents were purchased from New England Biolab (NEB, Massachusetts, USA). The reaction was conducted at 65 ºC for 1 h. After amplification, 3 µL of amplicon was added to 50 µL of 1 × PBS supplemented with 5% BSA, and applied to the test strip. After the sample flowed to the test strip, the detector mixture, which contained 2.5 µL of cyan anti-DIG, 5 µL of magenta anti-DNP, 5 µL of yellow anti-FITC and 5 µL of magenta anti-TAMRA in 25 µL of 1 × PBS supplemented with 10% BSA, was applied to the test strip. In the end, the test strip was washed with 50 µL of 1 × PBS supplemented with 5% BSA.

**Table 2. LAMP recipe**

| **Item** | **Initial Concentration** | **Final Concentration** | **25 µL Vol. (µL)** |
|---|---|---|---|
| 10x isothermal buffer | 10x | 1 × (2 mM Mg²⁺ final in 1 × buffer) | 2.5 |
| dNTP | 10 mM each | 1 mM each | 2.5 |
| MgSO₄, Mg²⁺ | 100mM | 8mM | 2 |
| Target Primers (18 primers) | 5 µM each | 200 nM each | 1 |
| Target DNA template | 1 µL of each of the 3 targets. Use water to make up the volume if the reaction contains < 3 targets. | | 3 |
| Control Primer | 10 µM each | 200 nM each | 0.5 |
| Control DNA template | 1 pg/µL. | 1 pg | 1 |
| Water | - | - | 11.5 |
| Warmstart Bst | 8 unit/µL | 8 unit | 1 |
| | | **Total Volume:** | 25 |

### APPLICATIONS

Paper or membrane-based diagnostic platform such as LFA is a wellestablished platform widely used in point-of-care diagnostics. A new strategy for multiplexing on diagnostic platform such as LFA has been disclosed herein. In addition to spatial separation, embodiments of the method disclosed herein employ morphology and composition-controlled Au/Ag nanoparticles of three primary colors to add another parameter for multiplexing. The colocalization of primary particles may result in an array of secondary colors as an indication of various target combinations. This two-parameter multiplexing strategy significantly improves the multiplexing capability of LFA, and extends the applicability of LFA to more complicated analysis. In the examples provided, using the proposed two-parameter multiplexing LFA, the inventors successfully identified all 32 combinations of two protein and three DNA targets. The inventors also successfully identified subtypes of three herpes viruses by amplifying the viral DNA with loopmediated isothermal amplification, and detecting the amplicons using the two-parameter multiplexing LFA.

Embodiments of the methods and related kits disclosed herein provide a highly multiplexed strategy of detecting different target analytes in a sample that is not only sensitive and specific, but also relatively equipment-free, easy to implement, convenient, low cost, and efficient, making them suitable point-of-care diagnostic or home diagnostic.

It will be appreciated by a person skilled in the art that other variations and/or modifications may be made to the specific embodiments. The present embodiments are, therefore, to be considered in all respects to be illustrative and not restrictive.

## Claims

1. An in vitro method of detecting the presence of different target analytes in a sample, the method comprising:
providing the sample and a plurality of reporter particles having ω different colours to a substrate, the substrate having α assigned spatial regions; and
determining, on the substrate, the presence of a colour arising from the reporter particles in each of α assigned spatial regions on the substrate,
wherein each of the assigned spatial regions on the substrate is configured to immobilize a target analyte or a group of target analytes that is different from the other assigned spatial regions,
wherein reporter particles of each different colour are configured to couple to a target analyte that is different from that of the reporter particles of the other different colours, and
wherein α is an integer that is at least two and ω is an integer that is at least three,
wherein the reporter particles are configured to give rise to a secondary colour when reporter particles of two or more different colours are colocalized in the same spatial region, wherein the secondary colour resulting from the colocalization of the reporter particles of two or more different colours from the ω different colours is distinct from another secondary colour resulting from the colocalization of reporter particles of other combinations of colours from the ω different colours, wherein the presence of a secondary colour arising from the colocalization of reporter particles in an assigned spatial region is indicative of the presence of at least two different target analytes in the sample.

2. The method of claim 1, wherein each of ω different colours is a colour selected from the group consisting of: a colour with absorbance wavelength of from 550 nm to 570 nm, a colour with absorbance wavelength of from 400 nm to 420 nm and a colour with absorbance wavelength of from 610 nm to 630 nm, optionally wherein each of ω different colours is a colour selected from the group consisting of: yellow, magenta and cyan, and when combined with one of more of the other ω different colours, is configured to give rise to a secondary colour selected from: blue, green, red and black.

3. The method of any one of the preceding claims, wherein the analyte comprises a polynucleotide and the method further comprises contacting the sample with a labelled oligonucleotide for hybridizing to at least a portion of the polynucleotide or its amplicons thereof, the labelled oligonucleotide being configured to couple to the reporter particles of at least one of ω different colours, optionally wherein the oligonucleotide comprises a primer.

4. The method of any one of the preceding claims, wherein the step of providing the sample and the plurality of reporter particles having ω different colours to a substrate comprises first providing the sample to the substrate and subsequently providing the plurality of reporter particles having ω different colours to the substrate.

5. The method of any one of the preceding claims, wherein the reporter particles of each of ω different colours have a single localized surface plasmon resonance (LSPR) absorption peak.

6. The method of any one of the preceding claims, wherein the reporter particles comprise one or more of the following selected from the group consisting of: solid gold nanoparticles, solid silver nanoparticles with gold core and hollow gold nanoshells.

7. The method of any one of the preceding claims, the method further comprising:
assigning a code to each spatial region based on the colour at the spatial region;
combining the code assigned to each spatial region to obtain a combined code, wherein the combined code is used to determine the presence of different target analytes in the sample; and optionally
comparing the combined code with a list of predetermined codes to determine the presence of different target analytes in the sample.

8. A kit for detecting different target analytes in a sample, the kit comprising:
a substrate that comprises α assigned spatial regions, each spatial region being configured to immobilize a target analyte or a group of target analytes that is different from the other assigned spatial regions; and
a plurality of reporter particles having ω different colours or precursors thereof, wherein the ω different colours are such that a secondary colour resulting from a combination of two or more of the ω different colours is distinct from other combinations of the ω different colours,
wherein the reporter particles of each different colour are configured to couple to a target analyte that is different from that of the reporter particles of the other different colours, and
wherein α is an integer that is at least two and ω is an integer that is at least three.

9. The kit of claim 8, wherein each of ω different colours is a colour selected from the group consisting of: a colour with absorbance wavelength of from 550 nm to 570 nm, a colour with absorbance wavelength of from 400 nm to 420 nm and a colour with absorbance wavelength of from 610 nm to 630 nm, optionally wherein each of ω different colours is a colour selected from the group consisting of: yellow, magenta and cyan, and when combined with one of more of the other ω different colours, is configured to give rise to a secondary colour selected from: blue, green, red and black.

10. The kit of any one of claims 8-9, further comprising an oligonucleotide for hybridizing to at least a portion of a polynucleotide of the target analyte, optionally wherein the oligonucleotide comprises a labelled oligonucleotide that is configured to couple to the reporter particles of at least one of ω different colours.

11. The kit of any one of claims 8-10, wherein the reporter particles of each of ω different colours have a single localized surface plasmon resonance (LSPR) absorption peak.

12. The kit of any one of claims 8-11, wherein the reporter particles comprise one or more of the following selected from the group consisting of: solid gold nanoparticles, solid silver nanoparticles with gold core, hollow gold nanoshells and any combinations thereof.

13. The kit of any one of claims 8-12, further comprising:
a list of predetermined codes as reference for determining the presence of different target analytes in the sample.

14. The method of any one of claim 1-7, wherein the different coloured reporter particles couple respectively to different target analytes to be immobilized at each of the assigned spatial regions.

15. The method or the kit of any one of claims 1-14, wherein each of ω different colours is distinguishable by unaided eyes.

## Patentansprüche

1. In-vitro-Verfahren zum Erfassen des Vorhandenseins verschiedener Zielanalyten in einer Probe, das Verfahren umfassend:
Bereitstellen der Probe und einer Vielzahl von Reporterpartikeln, die ω verschiedene Farben auf einem Substrat aufweisen, wobei das Substrat α zugeordnete räumliche Regionen aufweist; und
Bestimmen, auf dem Substrat, des Vorhandenseins einer Farbe, die aus den Reporterpartikeln in jeder von α zugeordneten räumlichen Regionen auf dem Substrat entsteht,
wobei jede der zugeordneten räumlichen Regionen auf dem Substrat konfiguriert ist, um einen Zielanalyten oder eine Gruppe von Zielanalyten zu immobilisieren, die von den anderen zugeordneten räumlichen Regionen verschieden ist,
wobei Reporterpartikel jeder verschiedener Farbe konfiguriert sind, um an einen Zielanalyten zu koppeln, der von dem der Reporterpartikel der anderen verschiedenen Farben verschieden ist, und
wobei α eine ganze Zahl ist, die mindestens zwei ist, und ω eine ganze Zahl ist, die mindestens drei ist,
wobei die Reporterpartikel konfiguriert sind, um eine Sekundärfarbe hervorzurufen, wenn Reporterpartikel von zwei oder mehr verschiedenen Farben in derselben räumlichen Region kolokalisiert werden, wobei die Sekundärfarbe, die aus der Kolokalisierung der Reporterpartikel von zwei oder mehr verschiedenen Farben aus den ω verschiedenen Farben resultiert, sich von einer anderen Sekundärfarbe eindeutig unterscheidet, die aus der Kolokalisierung von Reporterpartikeln anderer Kombinationen von Farben aus den ω verschiedenen Farben resultiert, wobei das Vorhandensein einer Sekundärfarbe, die aus der Kolokalisierung von Reporterpartikeln in einer zugeordneten räumlichen Region entsteht, auf Vorhandensein von mindestens zwei verschiedenen Zielanalyten in der Probe hinweist.

2. Verfahren nach Anspruch 1, wobei jede von ω verschiedenen Farben eine Farbe ist, die aus der Gruppe ausgewählt ist, bestehend aus: einer Farbe mit einer Absorptionswellenlänge von 550 nm bis 570 nm, einer Farbe mit einer Absorptionswellenlänge von 400 nm bis 420 nm und einer Farbe mit einer Absorptionswellenlänge von 610 nm bis 630 nm, optional wobei jede der ω verschiedenen Farben eine Farbe ist, die aus der Gruppe ausgewählt ist, bestehend aus: Gelb, Magenta und Cyan, und wenn diese mit einer oder mehreren der anderen ω verschiedenen Farben kombiniert wird, konfiguriert ist, um eine Sekundärfarbe hervorzurufen, die ausgewählt ist aus: Blau, Grün, Rot und Schwarz.

3. Verfahren nach einem der vorstehenden Ansprüche, wobei der Analyt ein Polynukleotid umfasst und das Verfahren ferner ein Inberührungbringen der Probe mit einem markierten Oligonukleotid zum Hybridisieren mit mindestens einem Anteil des Polynukleotids oder seiner Amplikons davon umfasst, wobei das markierte Oligonukleotid konfiguriert ist, um an die Reporterpartikel mindestens einer von ω verschiedenen Farben zu koppeln, optional wobei das Oligonukleotid einen Primer umfasst.

4. Verfahren nach einem der vorstehenden Ansprüche, wobei der Schritt des Bereitstellens der Probe und der Vielzahl von Reporterpartikeln, die ω verschiedene Farben aufweisen, zu einem Substrat zuerst das Bereitstellen der Probe zu dem Substrat und anschließend das Bereitstellen der Vielzahl von Reporterpartikeln umfasst, die ω verschiedene Farben aufweist, zu dem Substrat.

5. Verfahren nach einem der vorstehenden Ansprüche, wobei die Reporterpartikel von jeder von ω verschiedenen Farben ein einzelnes Absorptionsmaximum einer lokalisierten Oberflächenplasmonresonanz (localized surface plasmon resonance - LSPR) aufweisen.

6. Verfahren nach einem der vorstehenden Ansprüche, wobei die Reporterpartikel eines oder mehrere der Folgenden umfassen, die aus der Gruppe ausgewählt sind, bestehend aus: massiven Goldnanopartikeln, massiven Silbernanopartikeln mit Goldkern und hohlen Goldnanoschalen.

7. Verfahren nach einem der vorstehenden Ansprüche, das Verfahren ferner umfassend:
Zuordnen eines Codes zu jeder räumlichen Region basierend auf der Farbe an der räumlichen Region;
Kombinieren des Codes, der jeder räumlichen Region zugeordnet ist, um einen kombinierten Code zu erhalten, wobei der kombinierte Code verwendet wird, um das Vorhandensein verschiedener Zielanalyten in der Probe zu bestimmen; und optional
Vergleichen des kombinierten Codes mit einer Liste von zuvor bestimmten Codes, um das Vorhandensein verschiedener Zielanalyten in der Probe zu bestimmen.

8. Kit zum Erfassen verschiedener Zielanalyten in einer Probe, das Kit umfassend:
ein Substrat, das α zugeordnete räumliche Regionen umfasst, wobei jede räumliche Region konfiguriert ist, um einen Zielanalyten oder eine Gruppe von Zielanalyten zu immobilisieren, die von den anderen zugeordneten räumlichen Regionen verschieden ist; und
eine Vielzahl von Reporterpartikeln, die ω verschiedene Farben oder Vorläufer davon aufweisen, wobei die ω verschiedenen Farben derart sind, dass eine Sekundärfarbe, die aus einer Kombination von zwei oder mehr der ω verschiedenen Farben resultiert, sich von anderen Kombinationen der ω verschiedenen Farben eindeutig unterscheidet,
wobei die Reporterpartikel jeder verschiedenen Farbe konfiguriert sind, um an einen Zielanalyten zu koppeln, der von dem der Reporterpartikel der anderen verschiedenen Farben verschieden ist, und
wobei α eine ganze Zahl ist, die mindestens zwei ist, und ω eine ganze Zahl ist, die mindestens drei ist.

9. Kit nach Anspruch 8, wobei jede von ω verschiedenen Farben eine Farbe ist, die aus der Gruppe ausgewählt ist, bestehend aus: einer Farbe mit einer Absorptionswellenlänge von 550 nm bis 570 nm, einer Farbe mit einer Absorptionswellenlänge von 400 nm bis 420 nm und einer Farbe mit einer Absorptionswellenlänge von 610 nm bis 630 nm, optional wobei jede von ω verschiedenen Farben eine Farbe ist, die aus der Gruppe ausgewählt ist, bestehend aus: Gelb, Magenta und Cyan, und wenn diese mit einer oder mehreren der anderen ω verschiedenen Farben kombiniert werden, konfiguriert sind, um eine Sekundärfarbe hervorzurufen, die ausgewählt ist aus: Blau, Grün, Rot und Schwarz.

10. Kit nach einem der Ansprüche 8 bis 9, ferner umfassend ein Oligonukleotid zum Hybridisieren mit mindestens einem Anteil des Polynukleotids des Zielanalyts, optional wobei das Oligonukleotid ein markiertes Oligonukeotid umfasst, dass konfiguriert ist, um an die Reporterpartikel mindestens einer von ω verschiedenen Farben zu koppeln.

11. Kit nach einem der Ansprüche 8 bis 10, wobei die Reporterpartikel von jeder von ω verschiedenen Farben ein Absorptionsmaximum eines einzelnen lokalisierten Oberflächenplasmonresonanz (LSPR) aufweisen.

12. Kit nach einem der Ansprüche 8 bis 11, wobei die Reporterpartikel eines oder mehrere der Folgenden umfassen, die aus der Gruppe ausgewählt sind, bestehend aus: massiven Goldnanopartikeln, massiven Silbernanopartikeln mit Goldkern und hohlen Goldnanoschalen und beliebigen Kombinationen davon.

13. Kit nach einem der Ansprüche 8 bis 12, ferner umfassend:
eine Liste von zuvor bestimmten Codes als Referenz zum Bestimmen des Vorhandenseins verschiedener Zielanalyten in der Probe.

14. Verfahren nach einem der Ansprüche 1 bis 7, wobei die verschiedenen gefärbten Reporterpartikel jeweils an verschiedenen Zielanalyten koppeln, die in jeder der zugeordneten räumlichen Regionen zu immobilisieren sind.

15. Verfahren oder Kit nach einem der Ansprüche 1 bis 14, wobei jede von ω verschiedenen Farben mit bloßem Auge differenzierbar ist.

## Revendications

1. Procédé in vitro de détection de la présence de différents analytes cibles dans un échantillon, le procédé comprenant :
la fourniture de l'échantillon et d'une pluralité de particules rapporteuses ayant ω couleurs différentes à un substrat, le substrat ayant α régions spatiales attribuées ; et
la détermination, sur le substrat, de la présence d'une couleur provenant des particules rapporteuses dans chacune des α régions spatiales attribuées sur le substrat,
dans lequel chacune des régions spatiales attribuées sur le substrat est conçue pour immobiliser un analyte cible ou un groupe d'analytes cibles qui est différent des autres régions spatiales attribuées,
dans lequel des particules rapporteuses de chaque couleur différente sont conçues pour se coupler à un analyte cible qui est différent de celui des particules rapporteuses des autres couleurs différentes, et
dans lequel α est un nombre entier qui est au moins égal à deux et ω est un nombre entier qui est au moins égal à trois,
dans lequel les particules rapporteuses sont conçues pour donner lieu à une couleur secondaire lorsque des particules rapporteuses de deux couleurs différentes ou plus sont colocalisées dans la même région spatiale, dans lequel la couleur secondaire résultant de la colocalisation des particules rapporteuses de deux couleurs différentes ou plus provenant des ω couleurs différentes est distincte d'une autre couleur secondaire résultant de la colocalisation de particules rapporteuses d'autres combinaisons de couleurs provenant des ω couleurs différentes, dans lequel la présence d'une couleur secondaire résultant de la colocalisation de particules rapporteuses dans une région spatiale attribuée indique la présence d'au moins deux analytes cibles différents dans l'échantillon.

2. Procédé selon la revendication 1, dans lequel chacune des ω couleurs différentes est une couleur choisie dans le groupe constitué par : une couleur avec une longueur d'onde d'absorbance de 550 nm à 570 nm, une couleur avec une longueur d'onde d'absorbance de 400 nm à 420 nm et une couleur avec une longueur d'onde d'absorbance de 610 nm à 630 nm, éventuellement dans lequel chacune des ω couleurs différentes est une couleur choisie dans le groupe constitué par : jaune, magenta et cyan, et lorsqu'elle est combinée avec une ou plusieurs des autres ω couleurs différentes, est conçue pour donner lieu à une couleur secondaire choisie parmi : bleu, vert, rouge et noir.

3. Procédé selon l'une quelconque des revendications précédentes, dans lequel l'analyte comprend un polynucléotide et le procédé comprend en outre la mise en contact de l'échantillon avec un oligonucléotide marqué pour l'hybridation à au moins une partie du polynucléotide ou à ses amplicons, l'oligonucléotide marqué étant conçu pour se coupler aux particules rapporteuses d'au moins l'une des ω couleurs différentes, éventuellement dans lequel l'oligonucléotide comprend une amorce.

4. Procédé selon l'une quelconque des revendications précédentes, dans lequel l'étape de fourniture de l'échantillon et de la pluralité de particules rapporteuses ayant ω couleurs différentes à un substrat comprend d'abord la fourniture de l'échantillon au substrat et ensuite la fourniture de la pluralité de particules rapporteuses ayant ω couleurs différentes au substrat.

5. Procédé selon l'une quelconque des revendications précédentes, dans lequel les particules rapporteuses de chacune des ω couleurs différentes ont un seul pic d'absorption de résonance plasmonique de surface localisée (LSPR).

6. Procédé selon l'une quelconque des revendications précédentes, dans lequel les particules rapporteuses comprennent un ou plusieurs des éléments suivants choisis dans le groupe constitué par : des nanoparticules d'or solides, des nanoparticules d'argent solides avec un noyau d'or et des nanocoquilles d'or creuses.

7. Procédé selon l'une quelconque des revendications précédentes, le procédé comprenant en outre :
l'attribution d'un code à chaque région spatiale sur la base de la couleur au niveau de la région spatiale ;
la combinaison du code attribué à chaque région spatiale pour obtenir un code combiné, dans lequel le code combiné est utilisé pour déterminer la présence de différents analytes cibles dans l'échantillon ; et éventuellement
la comparaison du code combiné avec une liste de codes prédéterminés pour déterminer la présence de différents analytes cibles dans l'échantillon.

8. Kit pour la détection de différents analytes cibles dans un échantillon, le kit comprenant :
un substrat qui comprend α régions spatiales attribuées, chaque région spatiale étant conçue pour immobiliser un analyte cible ou un groupe d'analytes cibles qui est différent des autres régions spatiales attribuées ; et
une pluralité de particules rapporteuses ayant ω couleurs différentes ou leurs précurseurs, dans lequel les ω couleurs différentes sont telles qu'une couleur secondaire résultant d'une combinaison de deux des ω couleurs différentes ou plus est distincte d'autres combinaisons des ω couleurs différentes,
dans lequel les particules rapporteuses de chaque couleur différente sont conçues pour se coupler à un analyte cible qui est différent de celui des particules rapporteuses des autres couleurs différentes, et
dans lequel α est un nombre entier qui est au moins égal à deux et ω est un nombre entier qui est au moins égal à trois.

9. Kit selon la revendication 8, dans lequel chacune des ω couleurs différentes est une couleur choisie dans le groupe constitué par : une couleur avec une longueur d'onde d'absorbance de 550 nm à 570 nm, une couleur avec une longueur d'onde d'absorbance de 400 nm à 420 nm et une couleur avec une longueur d'onde d'absorbance de 610 nm à 630 nm, éventuellement dans lequel chacune des ω couleurs différentes est une couleur choisie dans le groupe constitué par : jaune, magenta et cyan, et lorsqu'elle est combinée avec une ou plusieurs des autres ω couleurs différentes, est conçue pour donner lieu à une couleur secondaire choisie parmi : bleu, vert, rouge et noir.

10. Kit selon l'une quelconque des revendications 8 à 9, comprenant en outre un oligonucléotide pour l'hybridation à au moins une partie d'un polynucléotide de l'analyte cible, éventuellement dans lequel l'oligonucléotide comprend un oligonucléotide marqué qui est conçu pour se coupler aux particules rapporteuses d'au moins l'une des ω couleurs différentes.

11. Kit selon l'une quelconque des revendications 8 à 10, dans lequel les particules rapporteuses de chacune des ω couleurs différentes ont un seul pic d'absorption de résonance plasmonique de surface localisée (LSPR).

12. Kit selon l'une quelconque des revendications 8 à 11, dans lequel les particules rapporteuses comprennent un ou plusieurs des éléments suivants choisis dans le groupe constitué par : des nanoparticules d'or solides, des nanoparticules d'argent solides avec un noyau d'or, des nanocoquilles d'or creuses et l'une quelconque de leurs combinaisons.

13. Kit selon l'une quelconque des revendications 8 à 12, comprenant en outre :
une liste de codes prédéterminés comme référence pour la détermination de la présence de différents analytes cibles dans l'échantillon.

14. Procédé selon l'une quelconque des revendications 1 à 7, dans lequel les différentes particules rapporteuses colorées se couplent respectivement à différents analytes cibles à immobiliser au niveau de chacune des régions spatiales attribuées.

15. Procédé ou kit selon l'une quelconque des revendications 1 à 14, dans lequel chacune des ω couleurs différentes peut être distinguée à l'œil nu.
